# EUROPÄISCHE PATENTSCHRIFT

(11) **EP 3 109 324 B1**
(45) Veröffentlichungstag und Bekanntmachung des Hinweises auf die Patenterteilung: **19.09.2018**
(21) Anmeldenummer: 15002200.2
(22) Anmeldetag: 24.07.2015
(51) Int. Cl.: C12Q 1/68

(54) **VERFAHREN ZUR DETEKTION VON CHROMOSOMENABERRATIONEN**
METHOD FOR DETECTING CHROMOSOME ABERRATIONS
PROCEDE DE DETECTION D'ABERRATIONS CHROMOSOMIQUES

(30) Priorität: 23.06.2015 EP 15001845; 13.07.2015 EP 15002075
(43) Veröffentlichungstag der Anmeldung: 28.12.2016
(73) Patentinhaber: ZytoVision GmbH, 27572 Bremerhaven (DE)
(72) Erfinder: Rogalla, Piere, 28816 Stuhr (DE); Hauke, Sven, 28203 Bremen (DE)
(74) Vertreter: Meissner Bolte Partnerschaft mbB

(56) Entgegenhaltungen:
- WO-A1-93/21345
- WO-A2-02/093130
- WO-A2-2005/111235
- US-B1- 6 344 315
- US-B1- 6 576 421
- D PRIMO ET AL: "Patterns of BCR/ABL gene rearrangements by interphase fluorescence in situ hybridization (FISH) in BCR/ABL+ leukemias: incidence and underlying genetic abnormalities", LEUKEMIA., Bd. 17, Nr. 6, 1. Juni 2003 (2003-06-01), Seiten 1124-1129, XP055297984, US ISSN: 0887-6924, DOI: 10.1038/sj.leu.2402963

## Beschreibung

Die vorliegende Erfindung betrifft das technische Gebiet der Nachweisverfahren für Chromosomenanomalien bzw. Chromosomenaberrationen.

Insbesondere betrifft die vorliegende Erfindung ein Verfahren zum Nachweis von mindestens zwei voneinander verschiedenen Chromosomenaberrationen mittels *In-Situ*-Hybridisierung. Weiterhin betrifft die vorliegende Erfindung eine zum Nachweis von mindestens zwei voneinander verschiedenen Chromosomenaberrationen geeignete Zusammensetzung sowie deren erfindungsgemäße Verwendungen. Weiterer Gegenstand der vorliegenden Erfindung sind zudem die Verwendungen von mindestens drei voneinander verschiedenen, mit Detektionslabeln markierten lokusspezifischen Hybridisierungssonden. Schließlich ist Gegenstand der vorliegenden Erfindung ein Kit zum Nachweis von Chromosomenaberrationen.

Vielen Tumorerkrankungen liegen strukturelle und numerische Chromosomenmutationen, wie Translokationen, Inversionen, segmentelle Duplikationen, Deletionen, Insertionen, Duplikationen, Aneuplodien und Amplifikationen, zu Grunde. Der Nachweis dieser Veränderungen als prädiktiver, prognostischer oder differentialdiagnostischer Marker erfolgt in der Regel durch *In-Situ-*Hybridisierungen (ISH).

Die *In-Situ-*Hybridisierung beruht auf der Hybridisierung bzw. Paarung komplementärer Basen von Nukleinsäure-Einzelsträngen, insbesondere DNA-Einzelsträngen, so dass spezifische Nukleinsäuresequenzen in einer Probe, insbesondere in einem Gewebe oder Zellpräparat, detektiert werden können. Dazu werden direkt oder indirekt markierte, synthetisch hergestellte Sonden mit Nukleinsäure-Einzelsträngen der Probe hybridisiert und anschließend detektiert.

Zu Detektionszwecken können unter anderem fluoreszenzmarkierte Nukleinsäurefragmente bzw. fluoreszenzmarkierte Hybridisierungssonden (Fluoreszente ISH (FISH)) zum Einsatz kommen. Darüber hinaus können antigenmarkierte Sonden, insbesondere Hapten-markierte Sonden, zum Einsatz kommen, welche anschließend mit Hilfe von Antikörpern durch Farbreaktionen sichtbar gemacht werden, so dass eine lichtmikroskopische Analyse möglich ist ((Hellfeld ISH (BrISH), Chromogene ISH (CISH), Silber ISH (SISH)).

Der Vorteil der FISH ist, dass multiple genomische Bereiche simultan und voneinander deutlich unterscheidbar nachgewiesen werden können. Hierzu werden Nukleinsäurefragmente, die unterschiedliche genomische Bereiche adressieren bzw. hierfür spezifisch sind, jeweils mit unterschiedlichen Fluoreszenzfarbstoffen, die sich in ihrem Absorptions- und/oder Emissionsspektrum voneinander unterscheiden, markiert bzw. gekoppelt. Werden solche mehrfarbigen Sonden, welche separate, unterschiedliche Einzelsonden umfassen, z. B. an Metaphase-Chromosomen-Präparaten oder an Interphase-Zellkern-Präparaten verwendet, so können die einzelnen Farben durch Verwendung spezifischer Mikroskop-Filter, welche genau definierte Wellenlängenbereiche des Lichts zur Anregung der Farbstoffe auf das Präparat leiten sowie genau definierte Wellenlängenbereiche des von den Farbstoffen emittierten Lichts zum Auswerter leiten, getrennt voneinander dargestellt werden (sogenannter Single-Bandpass-Filterset). Darüber hinaus existieren auch Filter bzw. Filtersets, welche die simultane Darstellung unterschiedlicher Fluoreszenzfarbstoffe und somit mehrerer Nukleinsäurefragmente erlauben. Bei zwei unterschiedlichen Fluoreszenzfarbstoffen spricht man beispielsweise von einem Dual-Bandpass-Filterset.

Der simultanen Darstellung sind jedoch deutliche Grenzen gesetzt, da je genomischer Region, die durch eine spezifische Sonde nachgewiesen wird, nur ein Label verwendet werden kann. Weiterhin liegen die Absorptions- und Emissionsbereiche der Farbstoffe häufig so nah beieinander, dass sie durch die Mikroskop-Filter-Sets nicht voneinander getrennt werden können. Aus diesen Gründen werden in der FISH simultan üblicherweise nur zwei Farben (orange/rot und grün) bzw. drei Farben (orange/rot und grün simultan bzw. gemeinsam mit einer blauen Kerngegenfärbung (DAPI)) analysiert. In etwa die gleichen Einschränkungen, die für die FISH darstellbar sind, gelten für die BrISH. Hier ist der Stand der Technik die Verwendung von zwei Haptenen, zumeist ausgewählt aus der Gruppe Biotin, Dinitrophenyl (DNP) und Digoxigenin, und zwei antikörpergekoppelten Enzymen, zumeist alkalische Phosphatase und Peroxidase.

Die genannten Einschränkungen bei der simultanen Darstellung bzw. Analyse haben einen maßgeblichen Einfluss auf die Zusammensetzung (Komposition) bzw. die Abstimmung der Sonden für den Nachweis von strukturellen und numerischen Chromosomenaberrationen, die zur Diagnostik an Tumoren mit sich in der Interphase befindlichen Zellen nur mit sogenannten lokusspezifischen Sonden durchgeführt werden können, wobei gelegentlich parallel zum Nachweis von numerischen Chromosomenmutationen auch sogenannte Repetitive-Sequenzen-spezifische Sonden verwendet werden.

Unter lokusspezifischen Sonden werden solche Sonden verstanden, welche ausgewählte DNA-Abschnitte eines Chromosom, zumeist einzelne Gene oder benachbarte Gene, mit einer Größe von insgesamt bis zu etwa 1.000 kb adressieren und als genspezifische Sonden oder als single copy-Sonden bezeichnet werden. Repetitive-Sequenzen-spezifischen Sonden sind Sonden, die repetitive Sequenzen adressieren und daher Regionen mit einer Größe von mehrerer 1.000 kb adressieren. Diese Sonden umfassen beispielweise auch Zentromer- oder Alpha-Satelliten-Sonden.

In Bezug auf die Detektion von Translokationen und Inversionen gibt es prinzipiell zwei maßgebliche Techniken und zugrundeliegende Sondenzusammensetzungen bzw. Sondenkompositionen: Das Prinzip des Entstehens von Fusionssignalen (sogenannte Dual-Color-Dual-Fusion-Ansätze) (WO 02/093130 A2) einerseits sowie des sich Auftrennens von Fusionssignalen (sogenannte Dual-Color-Break-Apart- bzw. Dual-Color-Split-Ansätze) andererseits. In der nachfolgenden Darstellung dieser beiden Prinzipien und in den abgeleiteten Signalmustern ist zu beachten, dass eine normale Zelle in der Regel diploid ist, d. h. jedes Allel zweifach vorliegt. Da von Aberrationen in der Regel jeweils nur eines der beiden Allele betroffen wird, ist neben dem aberranten Signal in der Regel auch noch das normale Signal des nicht von der Aberration betroffenen Allels sichtbar. Zum besseren Verständnis wird nachfolgend das Signalmuster des normalen Signals nicht immer explizit beschrieben.

Bei Dual-Color-Dual-Fusion-Ansätzen wird der Bereich eines ersten Bruchpunkts des Chromosoms proximal und distal von Nukleinsäurefragmenten der gleichen Farbe (z. B. orange) flankiert, der Bereich eines zweiten Bruchpunkts, d. h. des reziproken Translokationspartners, wird proximal und distal von Nukleinsäurefragmenten einer zweiten Farbe (z. B. grün) flankiert. Die normale Situation, d. h. ohne chromosomale Brüche im Bereich der beiden Translokationspartner, wird hierbei durch ein grünes und ein räumlich getrenntes oranges Signal charakterisiert.

Im Falle einer reziproken Translokation kommt es innerhalb der Bruchpunkte beider Translokationspartner zu Brüchen und der proximale Bereich des einen Translokationspartners fusioniert mit dem distalen Bereich des anderen Partners und vice versa. Es entstehen somit zwei grün/orange Signal-Paare, auch Fusionssignale genannt, da sich die unterschiedlich farbigen Signale oftmals überlappen. Der Nachteil dieser Sonden-Techniken ist, dass die Fusionssignale nur entstehen, wenn die Bruchpunkte beider Translokationspartner im Bereich der jeweiligen markierten Nukleinsäurefragmente liegen. Bei Translokationen, die nur einen der beiden Partner betreffen, entstehen keine Fusionssignale. Es kommt hierbei lediglich zur Entstehung eines zusätzlichen Signals mit der Farbe des Signals, welches charakteristisch für den durch die Translokation betroffenen Partner ist. Dies bedeutet, dass ein zusätzliches grünes Signal entsteht, wenn der Bruchpunkt der Translokation in dem Bereich lag, der von den mit grünem Fluorochrom markierten Nukleinsäuren überdeckt wurde. Nachteil dieser Sonden-Zusammensetzung ist, dass mit Hilfe der verwendeten zwei Farben nur die beiden Bruchpunktbereiche der gleichen Translokation bzw. Inversion markiert werden und somit nur eine spezifische Translokation bzw. Inversion detektiert werden kann.

Bei Dual-Color-Break-Apart-Ansätzen wird der Bereich eines Bruchpunkts proximal und distal von unterschiedlich markierten oder farbig-markierten Nukleinsäurefragmenten flankiert (z. B. distal orange, proximal grün). Die normale Situation, d. h. ohne chromosomalen Bruch dieses Bereiches, wird dabei durch ein Fusionssignal charakterisiert. In einer aberranten Situation, d. h. wenn es zu einem chromosomalen Bruch zwischen den Sondenfragmenten kommt, trennen sich die Signale räumlich voneinander. Der Unterschied zwischen der normalen Situation und der aberranten Situation ist also gekennzeichnet durch den Abstand der unterschiedlich farbigen Signale. Aussagen über beteiligte Translokationspartner sind mit diesem Verfahren nicht möglich. Es lässt lediglich den Schluss zu, dass eine spezifische chromosomale Umlagerung stattgefunden hat. Nachteil dieser Sonden-Zusammensetzung ist, dass mit Hilfe der verwendeten zwei Farben nur ein einziger Bruchpunktbereich und somit nur eine spezifische Translokation bzw. Inversion detektiert werden kann.

Was Deletionen, Aneuplodien und Amplifikationen anbelangt, wird üblicherweise nur eine maßgebliche Technik und zugrundeliegende Sondenzusammensetzung, die sich im Allgemeinen auf lokusspezifische Sonden bezieht, eingesetzt. Unter Umständen werden jedoch lokusspezifische Sonden auch durch sogenannte Repetitive-Sequenzen-spezifische Sonden, wie beispielsweise Zentromer- oder Alpha-Satelliten-Sonden, ergänzt: Das Prinzip des Nachweises von Zugewinn oder Verlust von Signalen durch das Auftreten von Deletionen, Aneuplodien und Amplifikationen erfolgt üblicherweise in sogenannten Dual-Color-Probe-Ansätzen. Auch bei diesem Prinzip, welches nachfolgend beschrieben wird, und den daraus abgeleite-Darüber hinaus werden im Stand der Technik dreifach-FISH-Ansätze beschrieben, welche den Nachweis von unterschiedlichen Translokationsereignissen adressieren, die in einer chromosomalen Region nebeneinander clustern können (d. h. es sind unterschiedliche Gene betroffen, die in Nachbarschaft liegen). Dabei werden für die entsprechende Auswertung der Signalmuster jeweils nur zwei Farben analysiert, die eine einzelne Aberration nachweisen, wobei die dritte Farbe jeweils keine Rolle spielt. Es werden drei unterschiedliche lokusspezifische Sonden verwendet, die jeweils mit einem anderen Label markiert sind.

Was die Hellfeld-ISH (BrISH) unter Verwendung von mehr als zwei Farben anbelangt, wird im Stand der Technik diesbezüglich die Durchführung einer chromogenen Dreifach-*In*-*Situ*-Hybridisierung beschrieben, die allgemein auf den Nachweis von drei repetitiven chromosomalen Bereichen abzielt. Nach dem derzeitigen Stand der Technik werden Translokationen mittels BrISH nur unter Verwendung von zwei Haptenen und somit zwei Farbstoffen nachgewiesen. In der Patentanmeldung WO 2012/150022 A1 wird ein Verfahren beschrieben, welches die Anwendung von drei unterschiedlichen Sonden mittels der BrISH-Methode unter Verwendung der drei Label Biotin, Digoxigenin und DNP, welche zu drei unterschiedlichen Farben führen, zur Detektion von Inversionen offenbart.

Die US 6 576 421 B1 beschreibt ein Verfahren zum Nachweis von mit Krebs assoziierten Translokationen bei dem drei Sonden verwendet werden, von denen zwei auf einer Seite eines Bruchpunktes auf einem Fusionschromosom hybridisieren und die dritte Sonde auf der anderen Seite des Bruchpunktes. Jede der Sonden ist mit einem Marker markiert, der sich von denen der anderen Sonden unterscheidet.

Die US 6 344 315 B1 offenbart Verfahren zur Färbung von Interphase-Chromosomenmaterial, bei denen eine Nukleinsäuresonde verwendet wird, die eine Länge von mehr als 50.000 Basen aufweist, und in denen zum Beispiel zur Durchführung eines FISH Verfahrens eine Biotin-markierte ABL Sonde verwendet wird, die indirekt mit dem Fluorochrom Texas Rot nachgewiesen wird, und eine Digoxigeninmarkierte BCR Sonde, die indirekt mit dem Fluorochrom FITC nachgewiesen wird.

Die WO 93/21345 A1 beschreibt Verfahren zum Nachweis einer Deletion eines Gens, bei denen zwei Sonden verwendet werden, die mit voneinander unterscheidbaren fluoreszenten Markern versehen sind.

Die WO 2005/111235 A2 beschreibt ein Verfahren, welches die Verwendung von drei Farben zu Detektionszwecken umfasst. Jedoch ist die chromosomale Region, die von dem dritten Label einer Sonde markiert wird, nicht unmittelbar von einer Veränderung betroffen, so dass bei einer Chromosomenstrukturänderung das erste Fusionssignal eliminiert wird, so dass ein neues Split Signal und ein neues Fusions-Darüber hinaus werden im Stand der Technik dreifach-FISH-Ansätze beschrieben, welche den Nachweis von unterschiedlichen Translokationsereignissen adressieren, die in einer chromosomalen Region nebeneinander clustern können (d. h. es sind unterschiedliche Gene betroffen, die in Nachbarschaft liegen). Dabei werden für die entsprechende Auswertung der Signalmuster jeweils nur zwei Farben analysiert, die eine einzelne Aberration nachweisen, wobei die dritte Farbe jeweils keine Rolle spielt. Es werden drei unterschiedliche lokusspezifische Sonden verwendet, die jeweils mit einem anderen Label markiert sind.

Was die Hellfeld-ISH (BrISH) unter Verwendung von mehr als zwei Farben anbelangt, wird im Stand der Technik diesbezüglich die Durchführung einer chromogenen Dreifach-*In*-*Situ*-Hybridisierung beschrieben, die allgemein auf den Nachweis von drei repetitiven chromosomalen Bereichen abzielt. Nach dem derzeitigen Stand der Technik werden Translokationen mittels BrISH nur unter Verwendung von zwei Haptenen und somit zwei Farbstoffen nachgewiesen. In der Patentanmeldung WO 2012/150022 A1 wird ein Verfahren beschrieben, welches die Anwendung von drei unterschiedlichen Sonden mittels der BrISH-Methode unter Verwendung der drei Label Biotin, Digoxigenin und DNP, welche zu drei unterschiedlichen Farben führen, zur Detektion von Inversionen offenbart.

Die WO 2005/111235 A2 beschreibt ein Verfahren, welches die Verwendung von drei Farben zu Detektionszwecken umfasst. Jedoch ist die chromosomale Region, die von dem dritten Label einer Sonde markiert wird, nicht unmittelbar von einer Veränderung betroffen, so dass bei einer Chromosomenstrukturänderung das erste Fusionssignal eliminiert wird, so dass ein neues Split Signal und ein neues Fusionssignal entstehen. Dieses Verfahren setzt Sonden ein, die jeweils nur mit einem Label markiert sind. Außerdem kann mit diesen Verfahren nur eine mögliche Translokation, welche durch die Sonden vorgegeben wird, nachgewiesen werden.

Die WO 02/093130 A3 offenbart ein Verfahren zur Detektion von chromosomalen Translokationen unter Verwendung von zwei oder alternativ vier Labeln bzw. Farbstoffen, welche jeweils distal und proximal die Bruchpunkte beider an einer Translokation beteiligen Bruchpunkte flankieren. Dieses Verfahren bietet keine Möglichkeiten, mehr als die eine Translokation an dem einen Bruchpunktbereich, der von den Sonden flankiert wird, nachzuweisen.

signal entstehen. Dieses Verfahren setzt Sonden ein, die jeweils nur mit einem Label markiert sind. Außerdem kann mit diesen Verfahren nur eine mögliche Translokation, welche durch die Sonden vorgegeben wird, nachgewiesen werden.

Die WO 02/093130 A3 offenbart ein Verfahren zur Detektion von chromosomalen Translokationen unter Verwendung von zwei oder alternativ vier Labeln bzw. Farbstoffen, welche jeweils distal und proximal die Bruchpunkte beider an einer Translokation beteiligen Bruchpunkte flankieren. Dieses Verfahren bietet keine Möglichkeiten, mehr als die eine Translokation an dem einen Bruchpunktbereich, der von den Sonden flankiert wird, nachzuweisen.

Insgesamt lässt sich somit feststellen, dass es mit den aus dem Stand der Technik bekannten Verfahren nicht möglich ist, gleichzeitig bzw. simultan in effizienter Weise mehrere voneinander verschiedene Chromosomenaberrationen in Zellen bzw. Geweben mittels *In-Situ*-Hybridisierung zu detektieren.

Eine simultane bzw. gleichzeitige Zuordnung definierter DNA- bzw. Chromosomenbereiche im Rahmen in *In-Situ*-Hybridisierungen ist bislang nur bei Verfahren zum Nachweis ganzer Chromosomen mit einer Größe beim Menschen zwischen etwa 50 Mbp und 250 Mbp oder großer chromosomaler Bereiche, z.B. Chromosomenarme, unter Verwendung von sogenannten Whole Chromosome Painting Probes (WCP) oder Partial Chromosome Painting Probes (PCP) bekannt. Mit den zugrunde liegenden Techniken, z. B. mFISH (multiplex FISH), SKY-FISH (spectral karyotyping), multicolor FISH, COBRA-FISH (COmbined Binary Ratio labeling FISH) oder auch 24-color-FISH, können unter Nutzung von etwa vier bis sieben verschiedenen Fluoreszenzfarbstoffen insgesamt vierundzwanzig verschiedene Chromosome Painting Probes markiert und unterschieden werden. In einem ähnlichen Verfahren können in einer sogenannten 42-color-FISH die Chromosomenarmspezifischen Sonden aller Chromosomen unterschiedlich markiert werden. Die vorgenannten Verfahren sind jedoch nur für Zellen, welche sich in der Metaphase befinden, geeignet. Die vorgenannten Verfahren sind nur möglich, da mit den darin verwendeten Sonden nur genomische/chromosomale Bereiche in Metaphasen zumindest im Wesentlichen ohne Überlagerung chromosomalen Materials ausgewertet werden können. Eine Analyse von Zellen in der Interphase, welche im Allgemeinen erst eine Analyse des genetischen Materials von soliden Tumoren erlaubt, ist mit derartigen Verfahren nicht möglich. Darüber hinaus können die dabei zum Einsatz kommenden diese Sonden relativ einfach nachgewiesen werden, da sie große Bereiche adressieren. Die Auswertung dieser Analysen kann nicht manuell, d.h. unter Betrachtung der Signale am Fluoreszenzmikroskop erfolgen, sondern nur computerbasiert mit Hilfe geeigneter Auswertesoftware.

Die derzeit im Stand der Technik bekannten Methoden und Sondenzusammensetzungen für BrISH und FISH im Zusammenhang mit strukturellen und numerischen Chromosomenmutationen bzw. Chromosomenaberrationen, insbesondere im Zusammenhang mit Tumoren bzw. Krebserkrankungen, sind mit gewissen Nachteilen verbunden. So existieren keine Kompositionen lokusspezifischer Sonden und Methoden, welche eine sichere, einfache und schnelle Detektion und Diskriminierung mehrerer potentieller unterschiedlicher struktureller und/oder numerischer Chromosomenmutationen bzw. Chromosomenaberrationen erlauben.

Insbesondere bei den strukturellen Chromosomenmutationen ist die simultane oder gleichzeitige Analyse mehrerer unterschiedlicher chromosomaler Mutationen, die sich nicht bedingen, d.h. kein chromosomales Material austauschen bzw. nicht reziprok sind, insbesondere in zugrunde liegenden Break-Apart-Ansätzen gar nicht oder nur sehr schwer möglich.

Der vorliegenden Erfindung liegt daher die Aufgabe zugrunde, ein Verfahren bzw. eine Zusammensetzung bereitzustellen, welches bzw. welche zur Detektion bzw. Analyse von Chromosomenmutationen bzw. Chromosomenaberrationen geeignet ist und die zuvor geschilderten Nachteile des Standes der Technik zumindest weitgehend vermeidet oder aber wenigstens abschwächt.

Insbesondere liegt der vorliegenden Erfindung die Aufgabe zugrunde, ein Verfahren bereitzustellen, welches einen zuverlässigen und simultanen Nachweis mehrerer voneinander verschiedener Chromosomenaberrationen, insbesondere voneinander unabhängiger (d.h. nicht reziproker) Chromosomenaberrationen, insbesondere in einem Ansatz ermöglicht. Gleichermaßen liegt der vorliegenden Erfindung die Aufgabe zugrunde, ein Verfahren bereitzustellen, welches darüber hinaus auch die Zuordnung von Chromosomenaberrationen zu einem bestimmten Chromosomen- bzw. DNA-Bereich ermöglicht.

Zur Lösung der zuvor geschilderten Aufgabe schlägt die vorliegende Erfindung ein Verfahren zum Nachweis von mindestens zwei voneinander verschiedenen Chromosomenaberrationen gemäß Patentanspruch 1 vor; weitere vorteilhafte Ausgestaltungen sind Gegenstand der diesbezüglich abhängigen Patentansprüche.

Weiterhin ist Gegenstand der vorliegenden Erfindung eine Zusammensetzung zum Nachweis von mindestens zwei voneinander verschiedenen Chromosomenaberrationen gemäß dem diesbezüglich unabhängigen Patentanspruch bzw. eine Zusammensetzung zur Verwendung bei der prophylaktischen bzw. therapeutischen Behandlung bzw. bei der Diagnose bzw. Prognose von im Zusammenhang mit Chromosomenaberrationen stehenden Erkrankungen.

Wiederum weiterer Gegenstand der vorliegenden Erfindung ist die Verwendung einer Zusammensetzung nach der vorliegenden Erfindung gemäß dem diesbezüglich unabhängigen Patentanspruch.

Weiterhin betrifft die vorliegende Erfindung die Verwendung von mindestens drei, vorzugsweise mindestens vier voneinander verschiedenen lokusspezifischen Hybridisierungssonden gemäß dem diesbezüglich unabhängigen Patentanspruch.

Weiterhin ist Gegenstand der vorliegenden Erfindung die Verwendung von mindestens einer, mit mindestens zwei Detektionslabeln markierten lokusspezifischen Hybridisierungssonde gemäß dem diesbezüglich unabhängigen Patentanspruch.

Schließlich ist Gegenstand der vorliegenden Erfindung ein Kit bzw. Kit-of-parts oder Set zum Nachweis von mindestens zwei voneinander verschiedenen Chromosomenaberrationen; weitere, vorteilhafte Eigenschaften sind Gegenstand des diesbezüglichen Unteranspruchs.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoff oder dergleichen stets 100% bzw. 100 Gew.-% resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Zur Lösung der zuvor geschilderten Aufgabe schlägt die vorliegende Erfindung - gemäß einem **ersten** erfindungsgemäßen Aspekt - ein Verfahren zum Nachweis von mindestens zwei voneinander verschiedenen Chromosomenaberrationen, insbesondere strukturellen und/oder numerischen Chromosomenaberrationen, vorzugsweise strukturellen Chromosomenaberrationen, mittels *In-Situ*-Hybridisierung durch Detektion von Chromosomen- und/oder DNA-Bereichen in einer biologischen Probe, vorzugsweise in einer oder mehreren Zelle(n) und/oder in einem oder mehreren Zellkern(en),
wobei die *In-Situ-*Hybridisierung als Interphase-*In-Situ*-Hybridisierung durchgeführt wird,
wobei die *In-Situ*-Hybridisierung mit mindestens vier voneinander verschiedenen, mit jeweils einem ersten Detektionslabel markierten, lokusspezifischen Hybridisierungssonden durchgeführt wird, wobei insbesondere zur Erzeugung mindestens eines Mischsignals mindestens eine der lokusspezifischen Hybridisierungssonden mit mindestens einem weiteren, von dem ersten Detektionslabel verschiedenen Detektionslabel, bezogen auf die jeweilige lokusspezifische Hybridisierungssonde, markiert ist, so dass ein Signalmuster erzeugt wird, und
wobei vorhandene Chromosomenaberrationen anhand des Signalmusters identifiziert und/oder einem Chromosomen- und/oder DNA-Bereich zugeordnet werden.

Mit anderen Worten liegt der vorliegenden Erfindung das Grundprinzip zugrunde, durch die zielgerichtete Erzeugung von Mischsignalen in durch Interphase-*In-Situ-*Hybridisierungen erzeugten Signalmustern die gleichzeitige bzw. simultane Detektion mehrerer, voneinander verschiedener Chromosomenaberrationen, insbesondere voneinander unabhängige, d.h. nicht reziproke Chromomenaberrationen, in einer biologischen Probe sowie deren Zuordnung zu einem detektierten Chromosomen- bzw. DNA-Bereichen zu ermöglichen.

Insbesondere kann es somit im Rahmen der vorliegenden Erfindung vorgesehen sein, dass es sich bei den Chromosomenaberrationen um voneinander unabhängige Chromosomenaberrationen handelt. Mit anderen Worten ausgedrückt kann es erfindungsgemäß vorgesehen sein, dass die Chromosomenaberrationen nicht voneinander abhängig sind. Gleichermaßen kann es vorgesehen sein, dass die Chromosomenaberrationen nicht reziprok sind.

Gemäß einer besonderen Ausführungsform der vorliegenden Erfindung ist es zudem bevorzugt, dass die Chromosomenaberrationen nicht mit reziproken bzw. voneinander abhängigen Chromosomenaberrationen im Zusammenhang stehen bzw. mit solchen assoziiert sind.

Die vorliegende Erfindung ist mit zahlreichen Vorteilen und Besonderheiten verbunden, welche nachfolgend in nicht beschränkender Weise diskutiert sind und als Indiz für die Patentfähigkeit der vorliegenden Erfindung zu werten sind.

Im Rahmen der vorliegenden Erfindung ist es insgesamt vollkommen überraschend gelungen, ein Verfahren zum Nachweis von Chromosomenaberrationen bereitzustellen, welches im Rahmen einer Interphase-*In-Situ*-Hybridisierung einerseits einen eindeutigen Nachweis mehrerer möglicher struktureller bzw. numerischer Chromosomenaberrationen und andererseits die eindeutige Unterscheidung dieser Chromosomenaberrationen bzw. eindeutige Zuordnung der detektierten Chromosomenaberrationen zu bestimmten Chromosomen- bzw. DNA-Bereichen in einem einzigen Hybridsierungsansatz erlaubt. Insbesondere kann es sich bei den Chromosomenaberrationen um voneinander unabhängige Chromosomenaberrationen handeln, welche sich nicht bedingen bzw. welche nicht reziprok sind. Dies war bislang im Stand der Technik, insbesondere im Rahmen von Interphase-*In-Situ-*Hybridisierungen, nicht möglich.

Mit dem erfindungsgemäßen Verfahren lassen sich somit auf Chromosomenaberrationen zu untersuchende insbesondere biologische Proben, wie beispielsweise Schnitte aus Geweben, insbesondere Tumorgeweben, deutlich schneller und effizienter analysieren, da eine einzelne Probe gleichzeitig bzw. in einem Ansatz auf mehrere, voneinander verschiedene Chromosomenaberrationen untersucht werden kann. Darüber hinaus können etwaige detektierte Chromosomenaberrationen einem definierten bzw. bestimmten DNA- bzw. Chromosomenbereich zugeordnet werden.

Weiterhin kann mit Hilfe des erfindungsgemäßen Verfahrens die für den Nachweis von Chromosomenaberrationen erforderliche Probenmenge signifikant reduziert werden. Dies ist insbesondere vor dem Hintergrund vorteilhaft, dass die Entnahme von Gewebe zu Untersuchungszwecken, insbesondere im Zusammenhang mit der Diagnose bzw. Erkennung oder weiterführenden Analyse von Krebserkrankungen, inzwischen üblicherweise mittels Feinnadelbiopsie erfolgt, welche nur die Entnahme einer begrenzten Probenmenge zulässt, wohingegen offene Biopsien, welche auch die Entnahme größerer Gewebemengen erlauben, zunehmend seltener durchgeführt werden.

Weiterhin wird aufgrund der hohen Effizienz des erfindungsgemäßen Verfahrens die erforderliche Menge an mitunter kostenintensiven Materialien, wie Enzymen, Fluoreszenzfarbstoffen und dergleichen, für die Durchführung der *In-Situ-*Hybridisierung reduziert, so dass das Verfahren auch im Hinblick auf ökonomische und ökologische Aspekte vorteilhaft ist.

Zum besseren Verständnis der vorliegenden Erfindung werden nachfolgend die zentralen Begrifflichkeiten und Bezeichnungen des erfindungsgemäßen Verfahrens definiert:
Erfindungsgemäß werden unter dem Begriff der Chromosomenaberrationen, synonym auch als Chromosomenanomalien bezeichnet, insbesondere strukturelle und numerische Chromosomenaberrationen verstanden. Bei strukturellen Chromosomenaberrationen liegen Veränderungen im Aufbau eines Chromosoms vor, so dass diese auch als Chromosomenmutation bezeichnet werden. Insbesondere kann es sich dabei um Inversionen, Translokationen, Deletionen, segmentelle Duplikationen, Insertionen, Duplikationen bzw. Amplifikationen handeln. Numerische Chromosomenaberrationen hingegen führen zu einer Veränderung der Chromosomenanzahl. Synonym wird auch die Bezeichnung Genommutation verwendet. Bei numerischen Chromosomenaberrationen bzw. Genommutationen kann es sich insbesondere um Aneuplodien bzw. Polyploidien handeln. Das erfindungsgemäße Verfahren ist insbesondere zur Detektion struktureller Chromosomenaberrationen geeignet.

Die erfindungsgemäß eingesetzte *In-Situ*-Hybridisierung beruht auf der Hybridisierung bzw. Paarung komplementärer Basen von Nukleinsäure-Einzelsträngen, insbesondere DNA-Einzelsträngen, so dass spezifische Nukleinsäuresequenzen in einer Probe, wie einem Gewebe oder einem Zellpräparat, detektiert werden können. Im Rahmen der *In-Situ*-Hybridisierung werden direkt oder indirekt markierte, synthetisch hergestellte insbesondere lokusspezifische Hybridisierungssonden mit Nukleinsäure-Einzelsträngen der Probe hybridisiert und anschließend detektiert.

Grundsätzlich kann die *In-Situ*-Hybridisierung zu verschiedenen Stadien des Zellzyklus der untersuchten Zellen bzw. Zellkerne stattfinden bzw. durchgeführt werden, wobei sich eine Durchführung in der Metaphase, wenn die Chromosomen im kondensierten Zustand vorliegen, oder in der Interphase, wenn die Chromosomen dekondensiert vorliegen, etabliert haben. Je nach Ziel bzw. Zweck der *In-Situ-*Hybridisierung ist eine Durchführung an kondensierten Chromosomen in der Metaphase nicht immer möglich, insbesondere beispielsweise bei der Untersuchung der Zellen solider Tumore auf Chromosomenaberrationen. Erfindungsgemäß ist es daher vorgesehen, die *In-Situ-*Hybridisierung an Zellen bzw. Zellkernen, welche sich in der Interphase befinden, durchzuführen.

Unter lokusspezifischen Hybridisierungssonden werden im Rahmen der vorliegenden Erfindung für einen bestimmten Chromosomenbereich bzw. DNA-Bereich spezifische bzw. zu einem bestimmten Chromosomenbereich bzw. DNA-Bereich des DNA-Materials bzw. des genetischen Materials in einer zu untersuchenden Probe komplementäre Sonden verstanden. Üblicherweise basieren die erfindungsgemäß verwendeten Hybridisierungssonden auf Nukleinsäuren bzw. Nukleinsäurefragmenten und sind imstande, an den zu detektierenden Chromosomenbereich bzw. DNA-Bereich spezifisch zu binden bzw. zu hybridisieren. Der zu detektierende Chromosomenbereich bzw. DNA-Bereich kann eine variable Länge aufweisen. Insbesondere kann es vorgesehen sein, dass ein zu detektierender Chromosomenbereich bzw. DNA-Bereich ein einziges bzw. ein einzelnes Gen teilweise oder vollständig umfasst. Gleichermaßen kann es auch vorgesehen sein, dass ein zu detektierender Chromosomenbereich bzw. DNA-Bereich mehrere Gene, vorzugsweise benachbarte Gene, bevorzugt zwei Gene, teilweise oder vollständig umfasst.

Was die erfindungsgemäße Ausgestaltung der Hybridisierungssonden im Speziellen anbelangt, kann es insbesondere vorgesehen sein, dass eine lokusspezifische Hybridisierungssonde auf mehreren, insbesondere einer Vielzahl von Nukleinsäurefragmenten (synonym auch Sondenfragmente) basiert, welche in ihrer Gesamtheit als eine lokusspezifische Hybridisierungssonde bezeichnet werden. Darüber hinaus ist es möglich - wenn auch weniger bevorzugt - dass die lokusspezifischen Hybridisierungssonden auf nur einem einzelnen Nukleinsäurefragment basieren bzw. durch ein einzelnes Nukleinsäurefragment gebildet werden.

Detektionslabel bezeichnen im Rahmen der vorliegenden Erfindung Stoffe bzw. Substanzen, welche zu Nachweis- bzw. Detektionszwecken an Nukleinsäuren bzw. Nukleinsäurefragmente, insbesondere Hybridisierungssonden, gekoppelt werden. Die Auswahl geeigneter Detektionslabel liegt im üblichen Können des Fachmanns und bedarf keiner weiteren Ausführungen an dieser Stelle. Die mit Detektionslabeln markierten und an den nachzuweisenden bzw. zu detektierenden DNA- bzw.

Chromosmenabschnitt mittels *In-Situ*-Hybridisierung gebundenen bzw. hybridisierten Nukleinsäurefragmente können durch dem Fachmann an sich bekannte und an die verwendeten Detektionslabel angepasste Verfahren direkt oder indirekt nachgewiesen werden, beispielsweise mittels Fluoreszenzmikroskopie bzw. nach insbesondere enzymatischer Umsetzung bzw. Sichtbarmachung mittels enzymatisch umgesetzter Farbstoffsubstrate durch Hellfeldmikroskopie. Insbesondere wird durch die Detektionslabel an den lokusspezifischen Hybridisierungssonden im Rahmen der *In-Situ*-Hybridisierung ein Signalmuster erzeugt, welches die Grundlage der Untersuchung einer Probe auf etwaige Chromosomenaberrationen dient.

Zudem bezieht sich der erfindungsgemäß verwendete Begriff "Detektionslabel" nachfolgend auf die Art bzw. den Typ von Detektionslabel und nicht auf die numerische Anzahl an Detektionslabel-Molekülen, d.h. Formulierungen wie "mindestens ein Detektionslabel" sind auf einen bestimmten Typ eines Detektionslabels bzw. die bestimmte Auswahl eines Detektionslabels gemeint ist. Der Begriff "mehrere Detektionslabel" bezieht sich somit ebenfalls auf die Auswahl voneinander verschiedener Detektionslabel unterschiedlichen Typs und nicht auf die eingesetzte Anzahl an Detektionslabel-Molekülen. Dass im Rahmen der Markierung von Hybridisierungssonden diese üblicherweise mit mehr als einem Detektionslabel-Molekül gekoppelt werden, versteht sich für den Fachmann von selbst.

Die erfindungsgemäß eingesetzten lokusspezifischen Hybridisierungssonden, insbesondere Sondenfragmente bzw. Nukleinsäurefragmente, hybridisieren somit spezifisch an einen ausgewählten DNA- bzw. Chromosomenbereich des genetischen Materials in einer Probe und erzeugen auf Basis der gekoppelten Detektionslabel im Rahmen der *In-Situ*-Hybridisierung ein Signalmuster. Unter einem Signalmuster wird im Rahmen der vorliegenden Erfindung die Gesamtheit aller durch die *In-Situ-*Hybridisierung erzeugten Signale auf Basis der mit Detektionslabeln markierten lokusspezifischen Hybridisierungssonden verstanden.

In diesem Zusammenhang hat sich im Rahmen der vorliegenden Erfindung überraschend gezeigt, dass durch die Markierung von lokusspezifischen Hybridisierungssonden mit mindestens zwei voneinander verschiedenen Detektionslabeln in dem Signalmuster gut detektierbare und darüber hinaus von den übrigen Signalen gut zu unterscheidende Mischsignale erzeugt werden können, welche die Zuordnung einer aufgetretenen Chromosomenaberration zu einem detektierten DNA- bzw. Chromosomenbereich erlauben. Bei einem Mischsignal im Sinne der Erfindung handelt es sich somit um ein Signal, welches durch mindestens zwei, aber gleichermaßen auch mehrere, sich auf einer lokusspezifischen Hybridisierungssonde befindende voneinander verschiedene Detektionslabel erzeugt wird. Da Mischsignale durch die mindestens zwei, insbesondere mehreren Detektionslabel einer lokusspezifischen Hybridisierungssonde erzeugt werden, sind diese auch im Falle von Chromosomenaberrationen in dem Signalmuster der *In-Situ-*Hybridisierung sichtbar bzw. bleiben auch im Falle von Chromosomenaberrationen bestehen. Mögliche Ausführungsformen bzw. Ausgestaltungen der lokusspezifischen Hybridisierungssonden zur Erzeugung von Mischsignalen sind nachfolgend noch detailliert erläutert.

Bevorzugte Ausführungsformen des erfindungsgemäßen Verfahrens werden nachfolgend im Detail geschildert:
Gemäß einer ersten erfindungsgemäßen Ausführungsform kann es vorgesehen sein, dass die ersten Detektionslabel der eingesetzten lokusspezifischen Hybridisierungssonden jeweils gleich sind. Gemäß einer zweiten, gleichermaßen bevorzugten erfindungsgemäßen Ausführungsform kann es vorgesehen sein, dass die eingesetzten lokusspezifischen Hybridisierungssonden mit jeweils voneinander verschiedenen ersten Detektionslabeln markiert sind.

Mit anderen Worten kann es erfindungsgemäß vorgesehen sein, dass die eingesetzten lokusspezifischen Hybridisierungssonden - rein exemplarisch und nicht beschränkend - als erstes Detektionslabel z.B. den gleichen Fluoreszenzfarbstoff oder das gleiche Hapten aufweisen.

Gleichermaßen können die eingesetzten lokusspezifischen Hybridisierungssonden beispielsweise - und ebenfalls nicht beschränkend - voneinander verschiedene Fluoreszenzfarbstoffe, voneinander verschiedene Haptenen oder dergleichen als jeweils erstes Detektionslabel aufweisen. Eine Markierung mit voneinander verschiedenen ersten Detektionslabeln hat sich insbesondere im Hinblick auf die Detektion von mit Chromosomenbrüchen einhergehenden Chromosomenaberrationen, wie Translokationen bzw. Inversionen, als vorteilhaft erwiesen.

Was darüber hinaus den erfindungsgemäßen Nachweis von Chromosomenaberrationen anbelangt, ist es erfindungsgemäß, dass mindestens zwei, insbesondere mehrere voneinander verschiedene Chromosomenaberrationen aus einer Vielzahl möglicher Chromosomenaberrationen in der Probe detektiert und/oder nachgewiesen wird bzw. werden.

Gleichermaßen kann es vorgesehen sein, dass mindestens zwei, insbesondere mehrere voneinander verschiedene Chromosomenaberrationen aus einer Vielzahl möglicher Chromosomenaberrationen gleichzeitig, insbesondere simultan, in der Probe nachgewiesen.

Gemäß einer weiteren bevorzugten Ausführungsform der vorliegenden Erfindung kann es vorgesehen sein, dass das erfindungsgemäße Verfahren als Multiplex-Verfahren zum gleichzeitigen Nachweis mehrerer, voneinander verschiedener Chromosomenaberrationen durchgeführt wird.

Besonderer Vorteil - wie zuvor bereits ausgeführt - des erfindungsgemäßen Verfahrens gegenüber den im Stand der Technik bekannten Verfahren zur Detektion von Chromosomenaberrationen mittels *In-Situ*-Hybridisierung liegt somit darin, dass nunmehr auch Proben, insbesondere auf Basis von sich in der Interphase befindenden Zellen bzw. Zellkernen, in einem einzelnen Hybridisierungsansatz simultan bzw. gleichzeitig auf mehrere etwaige Chromosomenaberrationen unter Zuordnung dieser zu einem bestimmten DNA- bzw. Chromosomenbereich untersucht werden können.

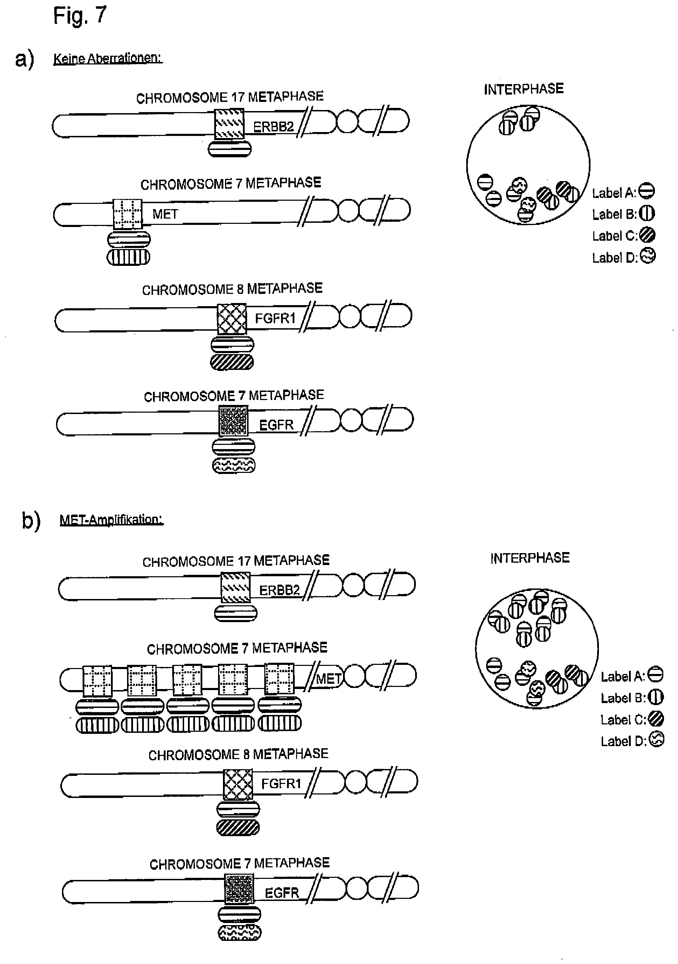

In diesem Zusammenhang kann es insbesondere vorgesehen sein, dass Chromosomenaberrationen in dem Signalmuster mittels des mindestens einen Mischsignals, insbesondere mehrerer Mischsignale, identifiziert bzw. den zu detektierenden Chromosomen- und/oder DNA-Bereichen zugeordnet werden. Diesbezüglich wird insbesondere auf Fig. 7 verwiesen, welcher exemplarisch der erfindungsgemäße Nachweis von Chromosomenaberrationen in Form von Amplifikationen zu entnehmen ist. Gemäß Fig. 7 werden vier verschiedene Chromosomenbereiche untersucht, wobei vier voneinander verschiedene Hybridisierungssonden, von denen drei zur Erzeugung jeweils spezifischer Mischsignale mit mindestens einem weiteren Detektionslabel markiert sind, eingesetzt werden (vgl. Fig. 7 a)). Auf Basis der Markierung von drei der vier Chromosomenbereiche mit einem jeweils spezifischen Mischsignal kann das durch eine Amplifikation eines detektierten Chromosomenbereichs erzeugte "Cluster" in dem Signalmuster einer Hybridisierungssonde bzw. einem detektierten Chromosomenbereich zugeordnet werden (vgl. Fig. 7 b)).

Erfindungsgemäß kann es somit insbesondere vorgesehen sein, dass die Markierung weiterer lokusspezifischer Hybridisierungssonden mit dem mindestens einen weiteren Detektionslabel derart erfolgt, dass die mit mindestens einem weiteren Detektionslabel markierten lokusspezifischen Hybridisierungssonden jeweils voneinander verschiedene Mischsignale in dem Signalmuster erzeugen.

Gleichermaßen kann es vorgesehen sein - insbesondere für den Fall, dass Chromosomenaberrationen, welche nicht aus Chromosomenbrüchen resultieren, wie Amplifikationen wie Deletionen, nachgewiesen werden sollen -, dass die Markierung weiterer lokusspezifischer Hybridisierungssonden mit mindestens einem weiteren Detektionslabel derart erfolgt, dass durch jede, mit mindestens einem weiteren Detektionslabel markierte lokusspezifische Hybridisierungssonde in dem Signalmuster ein für einen Chromosomen- und/oder DNA-Bereich spezifisches Mischsignal erzeugt wird. In diesem Zusammenhang ist es im Rahmen der vorliegenden Erfindung somit bevorzugt, wenn jeder durch eine lokusspezifische Hybridisierungssonde detektierte Bereich einem spezifischen Signal, insbesondere einem Mischsignal, innerhalb des Signalmusters zugeordnet werden kann.

Weiterhin ist es erfindungsgemäß bevorzugt, wenn die Markierung weiterer lokusspezifischer Hybridisierungssonden mit dem mindestens einen weiteren Detektionslabel derart erfolgt, dass jeder nachzuweisenden Chromosomenaberration in dem Signalmuster anhand eines spezifischen Mischsignals ein detektierter Chromosomen- und/oder DNA-Bereich zugeordnet wird.

Was die Anzahl der mit mindestens einem weiteren Detektionslabel markierten, lokusspezifischen Hybridisierungssonden anbelangt, so ist diese variabel und hängt insbesondere von der Anzahl der nachzuweisenden bzw. zu untersuchenden Chromosomenaberrationen ab:
Erfindungsgemäß ist es bevorzugt, wenn mindestens zwei, insbesondere mindestens drei, vorzugsweise mindestens vier, bevorzugt mindestens fünf, besonders bevorzugt mindestens sechs, ganz besonders bevorzugt mindestens sieben, weitere lokusspezifische Hybridisierungssonden mit mindestens einem weiteren, von dem ersten verschiedenen Detektionslabel markiert sind. Gleichermaßen kann es vorgesehen sein, dass durch mindestens zwei, insbesondere mindestens drei, vorzugsweise mindestens vier, bevorzugt mindestens fünf, besonders bevorzugt mindestens sechs, ganz besonders bevorzugt mindestens sieben, weitere lokusspezifische Hybridisierungssonden in dem Signalmuster jeweils für einen Chromosomen- und/oder DNA-Bereich spezifische Mischsignale erzeugt werden.

Diese zuvor geschilderte erfindungsgemäße Vorgehensweise ist insbesondere für den Fall dass Chromosomenaberrationen, welche nicht aus Chromosomenbrüchen resultieren, wie Amplifikationen oder Deletionen, nachgewiesen werden sollen, geeignet. Auf Basis einer Erhöhung der Anzahl von mit mindestens einem weiteren, vorzugsweise mehreren Detektionslabeln markierten Hybridisierungssonden, welche jeweils ein individuelles Mischsignal erzeugen, lässt sich somit auch die Zahl an gleichzeitig zu detektierenden bzw. nachzuweisenden Chromosomenaberrationen erhöhen.

Gemäß einer weiteren, speziellen Ausführungsform der vorliegenden Erfindung ist es zudem möglich, Chromosomenaberrationen, welche aus Chromosomenbrüchen resultieren, wie z.B. Translokationen oder Inversionen, zu detektieren und einem bestimmten Chromosomen- bzw. DNA-Bereich zuzuordnen:
Gemäß dieser erfindungsgemäßen Ausführungsform kann es vorgesehen sein, dass jeweils zwei lokusspezifische Hybridisierungssonden einen Chromosomenabschnitt, insbesondere Bruchpunktbereich, flankieren, wobei die jeweils einen Chromosomenabschnitt, insbesondere Bruchpunktbereich, flankierenden lokusspezifischen Hybridisierungssonden mit voneinander verschiedenen Detektionslabeln markiert sind, so dass durch die jeweils einen Chromosomenabschnitt, insbesondere Bruchpunktbereich, flankierenden lokusspezifischen Hybridisierungssonden in dem Signalmuster ein Fusionssignal erzeugt wird, insbesondere für den Fall, dass keine Chromosomenaberration vorliegt.

Mit anderen Worten ist es gemäß dieser Ausführungsform der vorliegenden Erfindung vorgesehen, dass sich die zu detektierenden DNA- bzw. Chromosomenbereiche distal und proximal von spezifisch ausgewählten Chromosomenabschnitten, insbesondere potentiellen Bruchpunktbereichen auf einem Chromosom, befinden. Dabei kann es insbesondere vorgesehen sein, dass entweder der distal oder aber der proximal gelegene Chromosomenabschnitt mit einer mit mindestens einem weiteren, von dem ersten Detektionslabel verschiedenen Detektionslabel markierten Hybridisierungssonde detektiert wird.

Unter einem Bruchpunktbereich werden im Rahmen der vorliegenden Erfindung diejenigen Bereiche eines Chromosoms verstanden, welche von Chromosomenbrüchen betroffen sein können. In Folge von Chromosomenbrüchen kann es zu Chromosomenaberrationen auf Basis struktureller Umlagerungen kommen, insbesondere zu Translokationen bzw. Inversionen. Für eine Reihe von Erkrankungen sind jeweils erkrankungsspezifische, auf Chromosomenbrüchen, insbesondere Translokationen bzw. Inversionen, beruhende Chromosomenaberrationen bekannt. Mit Hilfe des erfindungsgemäßen Verfahrens können somit mehrere, insbesondere bekannte Bruchpunktbereiche in dem genetischen Material einer Probe, insbesondere einer Gewebeprobe, auf das Vorliegen von Chromosomenaberrationen untersucht werden.

Bei dieser erfindungsgemäßen Ausführungsform wird somit durch die beiden, jeweils einen Bruchpunktbereich flankierenden lokusspezifischen Hybridisierungssonden ein Fusionssignal erzeugt, welches auf Basis der beiden voneinander verschiedenen Detektionslabel der ersten und zweiten lokusspezifischen Hybridisierungssonde erzeugt wird, insbesondere für den Fall, dass keine Chromosomenaberration vorliegt

Im Unterschied zu den zuvor bereits beschriebenen Mischsignalen, welche durch verschiedene Detektionslabel einer lokusspezifischen Hybridisierungssonde erzeugt werden, werden Fusionssignale durch voneinander verschiedene lokusspezifische Hybridisierungssonde erzeugt, welche in unmittelbarer Nähe voneinander an das genetische Material bzw. die DNA in der Probe hybridisiert vorliegen.

Mit anderen Worten wird somit im Rahmen des erfindungsgemäßen Verfahrens ein Fusionssignal erzeugt, für den Fall, dass an einem flankierten Bruchpunkt keine Chromosomenaberration vorliegt und die eingesetzten lokusspezifischen Hybridisierungssonden in unmittelbarer Nähe mit dem genetischen Material bzw. der DNA in der Probe hybridisieren. Liegt hingegen im Bereich eines Bruchpunkts eine Chromosomenaberration vor, können die eingesetzten lokusspezifischen Hybridisierungssonden aufgrund der strukturellen Umlagerung eines DNA-Abschnitts nicht mehr in unmittelbarer Nähe binden. Anstelle eines Fusionssignals dann werden in dem Signalmuster zwei Einzelsignale (synonym auch "Split-Signal"), welche vorzugsweise voneinander verschieden sind, detektiert.

Darüber hinaus erzeugen die mit mindestens einem weiteren, von dem ersten Detektionslabel verschiedenen Detektionslabel markierten Hybridisierungssonden Mischsignale auf Basis des ersten Detektionslabels und des mindestens einen weiteren Detektionslabels der jeweiligen Hybridisierungssonde. Diese können mit einer zweiten, einen Chromosomenabschnitt, insbesondere Bruchpunktbereich, flankierenden Hybridisierungssonde in dem Signalmuster somit ein Misch- und Fusionssignal ausbilden, für den Fall, dass keine Chromosomenaberration vorliegt. Im Falle von Chromosomenaberrationen hingegen werden auf Basis der in normalen Zellen bzw. Zellkernen einen Chromosomenabschnitt, insbesondere einen Bruchpunktbereich, flankierenden Hybridisierungssonden, von denen eine mit mindestens einem weiteren Detektionslabel markiert ist, ein Einzelsignal sowie ein Einzelsignal, welches von einem Mischsignal begleitet wird, in dem Signalmuster erzeugt.

Im Rahmen der vorliegenden Erfindung ist es somit möglich, anhand der Mischsignale auf Basis der mit mindestens einem weiteren Detektionslabel markierten lokusspezifischen Hybridisierungssonden Chromosomenaberrationen definierten Bruchpunktbereichen bzw. detektierten Chromosomen- bzw. DNA-Bereichen zuzuordnen.

Erfindungsgemäß ist es somit bevorzugt, wenn jeweils die mit mindestens einem weiteren, von dem ersten verschiedenen Detektionslabel markierte Hybridisierungssonde mit der zweiten einen Chromosomenabschnitt, insbesondere Bruchpunktbereich, flankierenden lokusspezifischen Hybridisierungssonde in dem Signalmuster ein Misch- und Fusionssignal erzeugt, insbesondere für den Fall, dass keine Chromosomenaberration vorliegt.

Darüber hinaus kann es vorgesehen sein, dass die mit mindestens einem weiteren, von dem ersten verschiedenen Detektionslabel markierte Hybridisierungssonde und die zweite einen Chromosomenabschnitt, insbesondere Bruchpunktbereich, flankierende lokusspezifische Hybridisierungssonde in dem Signalmuster jeweils ein Einzelsignal erzeugen, insbesondere wobei die mit mindestens einem weiteren Detektionslabel markierte Hybridisierungssonde in dem Signalmuster weiterhin ein Mischsignal erzeugt, insbesondere für den Fall, dass eine Chromosomenaberration vorliegt.

Insbesondere kann es im Rahmen des erfindungsgemäßen Verfahrens somit vorgesehen sein, dass in dem Signalmuster Chromosomenaberrationen durch Mischsignale einem detektierten Chromosomen- und/oder DNA-Bereich und/oder einem Chromosomenabschnitt, insbesondere Bruchpunktbereich, zugeordnet werden.

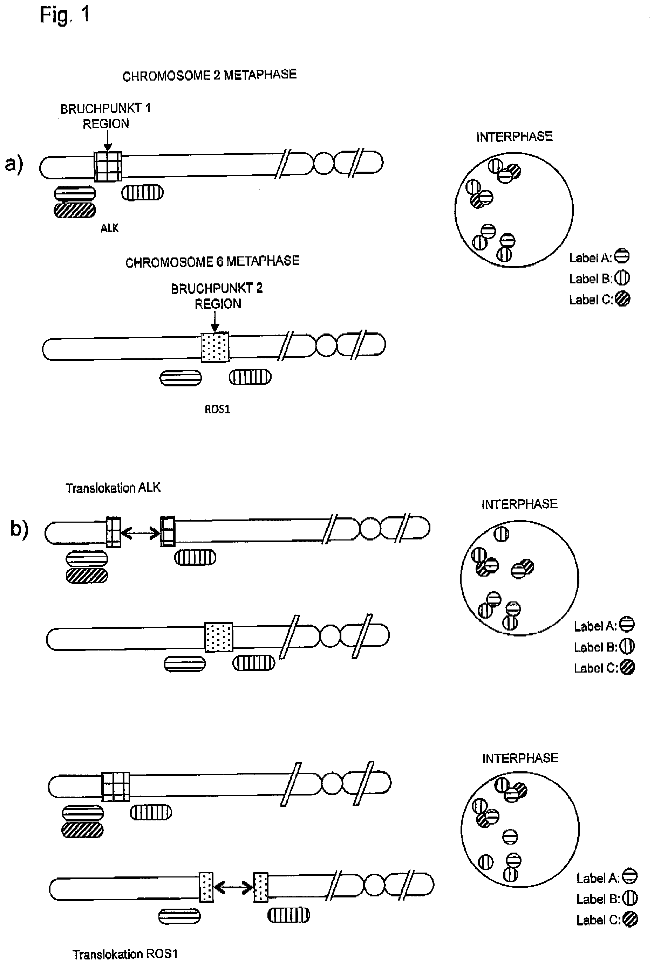

Gemäß dieser bevorzugten Ausführungsform des erfindungsgemäßen Verfahrens ist es somit möglich, eine Reihe von potentiellen Bruchpunktbereichen mittels Interphase-*In-Situ*-Hybridisierung simultan in einer einzelnen Probe darzustellen bzw. zu markieren. Für den Fall, dass keine Chromosomenaberration an den markierten Bruchpunkten stattgefunden hat, werden in dem Signalmuster Fusionssignale erzeugt, wohingegen das Auftreten von Einzelsignalen bzw. Split-Signalen das Vorliegen von Chromosomenaberrationen aufzeigt. Auf Basis der zusätzlich erzeugten Mischsignale können aberrante Einzelsignale bzw. Split-Signale einer bestimmten Hybridisierungssonde und somit einem bestimmten Bruchpunktbereich zugeordnet werden (vgl. Fig. 1).

Insbesondere kann es im Rahmen der vorliegenden Erfindung vorgesehen sein, dass mindestens sechs, vorzugsweise mindestens acht, bevorzugt mindestens zehn, besonders bevorzugt mindestens zwölf, noch mehr bevorzugt mindestens vierzehn verschiedene lokusspezifische Hybridisierungssonden eingesetzt werden, wobei jeweils zwei lokusspezifische Hybridisierungssonden jeweils einen Chromosomenabschnitt, insbesondere Bruchpunktbereich, flankieren bzw. dass höchstens vierundzwanzig verschiedene lokusspezifische Hybridisierungssonden eingesetzt werden, wobei jeweils zwei lokusspezifische Hybridisierungssonden jeweils einen Chromosomenabschnitt, insbesondere Bruchpunktbereich, flankieren.

Zudem ist er erfindungsgemäß bevorzugt, wenn die Markierung weiterer lokusspezifischer Hybridisierungssonden mit mindestens einem weiteren, von dem ersten verschiedenen Detektionslabel derart erfolgt, dass in dem Signalmuster anhand von Fusions- und Mischsignalen jeder flankierte Chromosomenabschnitt, insbesondere Bruchpunktbereich, und/oder jeder zu detektierende Chromosomen- und/oder DNA-Bereich identifiziert und/oder zugeordnet werden kann.

In diesem Zusammenhang ist es erfindungsgemäß insbesondere bevorzugt, wenn ein erster zu untersuchender Bruchpunktbereich von zwei Hybridisierungssonden, welche jeweils nur ein Detektionslabel aufweisen, flankiert wird, so dass dieser Bruchpunktbereich in dem Signalmuster lediglich durch ein Fusionssignal bzw. zwei Einzel- oder Split-Signale sichtbar ist. Jedem weiteren zu untersuchenden Bruchpunktbereich hingegen wird durch die Markierung von mindestens einer der beiden flankierenden Hybridisierungssonden mit mindestens einem weiteren Detektionslabel in dem Signalmuster ein spezifisches bzw. individuelles Mischsignal zugeordnet, so dass in dem Signalmuster insgesamt eine Vielzahl von Chromosomen- bzw. DNA-Bereichen, insbesondere Bruchpunktbereichen, voneinander unterscheidbar dargestellt werden kann.

Gemäß einer besonders bevorzugten Ausführungsform des erfindungsgemäßen Verfahrens kann es vorgesehen sein, dass
(a) eine mit einem Detektionslabel A markierte erste lokusspezifische Hybridisierungssonde und eine mit einem Detektionslabel B markierte zweite lokusspezifische Hybridisierungssonde einen Chromosomenabschnitt, insbesondere Bruchpunktbereich, flankieren und in dem mittels *In-Situ*-Hybridisierung erzeugten Signalmuster ein Fusionssignal A-B erzeugen,
(b) 2 bis 12 weitere lokusspezifische Hybridisierungssonden bis zu sechs weitere Chromosomenabschnitte, insbesondere Bruchpunktbereiche, flankieren, wobei ebenfalls jeweils eine der beiden einen Chromosomenabschnitt, insbesondere Bruchpunktbereich, flankierenden lokusspezifischen Hybridisierungssonden mit einem Detektionslabel A markiert ist und jeweils eine der beiden einen Chromosomenabschnitt, insbesondere Bruchpunktbereich, flankierenden lokusspezifischen Hybridisierungssonden mit einem Detektionslabel B markiert ist, so dass die jeweils einen Chromosomenabschnitt, insbesondere Bruchpunktbereich, flankierenden lokusspezifischen Hybridisierungssonden in dem mittels *In-Situ*-Hybridisierung erzeugten Signalmuster ein Fusionssignal A-B erzeugen und
(c) mindestens eine, vorzugsweise mehrere der lokusspezifischen Hybridisierungssonden mit mindestens einem weiteren Detektionslabel X markiert sind, so dass die mit mindestens einem weiteren Detektionslabel markierten lokusspezifischen Hybridisierungssonden in dem mittels *In-Situ-*Hybridisierung erzeugten Signalmuster Fusions- und Mischsignale A-B/X erzeugen,
wobei sich in dem mittels *In-Situ*-Hybridisierung erzeugten Signalmuster die Fusions- und Mischsignale A-B/X bei Chromosomenaberrationen zu Mischsignalen A/X und/oder B/X verändern und/oder
wobei sich in dem mittels *In-Situ*-Hybridisierung erzeugten Signalmuster die Fusionssignale A-B bei Chromosomenaberrationen zu Einzelsignalen A und/oder B verändern,
so dass Chromosomenaberrationen anhand des mittels der *In-Situ*-Hybridisierung erzeugten Signalmusters einem Chromosomen- und/oder DNA-Bereich und/oder einem durch zwei lokusspezifische Hybridisierungssonden flankierten Chromosomenabschnitt, insbesondere Bruchpunktbereich, zugeordnet werden.

Was darüber hinaus das in der zuvor beschriebenen Ausführungsform vorgesehene Detektionslabel X anbelangt, kann dieses durch ein einzelnes Detektionslabel, insbesondere ein Detektionslabel X₁, gebildet sein.

Darüber hinaus kann es vorgesehen sein, dass das Detektionslabel X durch mehrere, voneinander verschiedene Detektionslabel gebildet ist, vorzugsweise ausgewählt aus der Gruppe der Detektionslabel X₁, X₂, ... und/oder Xₙ, wobei der Index "n" eine natürliche ganze Zahl von 1 bis 20, insbesondere 1 bis 10, vorzugsweise 1 bis 5, darstellt. Diesbezüglich kann es zudem vorgesehen sein, dass die Detektionslabel X₁. X₂, ... und/oder Xₙ zur Erzeugung von voneinander verschiedenen, insbesondere spezifischen Mischsignalen in unterschiedlichen Verhältnissen zueinander eingesetzt werden.

Gemäß einer weiteren Ausführungsform ist es auch möglich, dass das Detektionslabel X durch mehrere, voneinander verschiedene Detektionslabel gebildet ist, vorzugsweise ausgewählt aus der Gruppe der Detektionslabel X₁, X₂, X₃, X₄, X₅ und/oder X₆. In diesem Zusammenhang ist es bevorzugt, wenn die Detektionslabel X₁, X₂, X₃, X₄, X₅ und/oder X₆ zur Erzeugung von voneinander verschiedenen, insbesondere spezifischen Mischsignalen in unterschiedlichen Verhältnissen zueinander eingesetzt werden.

Im Rahmen der vorliegenden Erfindung ist es somit überraschend gelungen, dass selbst auf Basis weniger einzelner Detektionslabel eine Vielzahl von spezifischen Mischsignalen erzeugt werden kann, indem zur Erzeugung von Mischsignalen die lokusspezifischen Hybridisierungssonden mit mindestens einem weiteren Detektionslabel zur Erzeugung von Mischsignalen mit mehreren, voneinander verschiedenen Detektionslabeln markiert werden.

Insbesondere kann es erfindungsgemäß vorgesehen sein, dass zur Erzeugung von Mischsignalen im Rahmen der Markierung von lokusspezifischen Hybridisierungssonden mehrere, insbesondere zwei bis sechs, voneinander verschiedene Detektionslabel in jeweils voneinander verschiedenen (Mengen)Verhältnissen eingesetzt werden. Beispielsweise - und keinesfalls beschränkend - kann eine lokusspezifische Hybridisierungssonde - neben dem ersten Detektionslabel - drei weitere Detektionslabel aufweisen, wobei dabei der Anteil des ersten Detektionslabels 20 %, der Anteil des zweiten Detektionslabels 60 % und der Anteil des dritten Detektionslabels 20 % beträgt, bezogen auf die drei weiteren Detektionslabel.

Gemäß einer wiederum weiteren Ausführungsform der vorliegenden Erfindung kann es zudem vorgesehen sein, dass
(a) eine mit einem Detektionslabel A markierte erste lokusspezifische Hybridisierungssonde und eine mit einem Detektionslabel B markierte zweite lokusspezifische Hybridisierungssonde einen ersten Chromosomenabschnitt, insbesondere ersten Bruchpunktbereich, flankieren und in dem mittels *In-Situ-*Hybridisierung erzeugten Signalmuster ein Fusionssignal A-B erzeugen,
(b) eine mit einem Detektionslabel A markierte dritte lokusspezifische Hybridisierungssonde und eine mit einem Detektionslabel B markierte vierte lokusspezifische Hybridisierungssonde einen zweiten Chromosomenabschnitt, insbesondere zweiten Bruchpunktbereich, flankieren und in dem mittels *In-Situ-*Hybridisierung erzeugten Signalmuster ein Fusionssignal A-B erzeugen, und (c) die dritte lokusspezifische Hybridisierungssonde und/oder die vierte lokusspezifische Hybridislerungssonde mit einem weiteren Detektionslabel X₁ markiert sind und in dem mittels *In-Situ*-Hybridisierung erzeugten Signalmuster Fusions- und Mischsignale A-B/X₁ erzeugen,
wobei in dem Signalmuster Chromosomenaberrationen des ersten Chromosomenabschnitts, insbesondere ersten Bruchpunktbereichs, durch Einzelsignale A und/oder B identifiziert werden und/oder wobei in dem Signalmuster Chromosomenaberrationen des zweiten Chromosomenabschnitts, insbesondere zweiten Bruchpunktbereichs, durch Mischsignale A/X₁ und/oder B/X₁ identifiziert und/oder dem zweiten Chromosomenabschnitt, insbesondere dem zweiten Bruchpunktbereich, zugeordnet werden.

Was die Vorgehensweise zur Analyse von Chromosomenaberrationen in dem mittels der *In-Situ*-Hybridisierung erzeugten Signalmuster anbelangt, so hat es sich als besonders effizient erwiesen, wenn in einem ersten Schritt die durch die ersten Detektionslabel erzeugten Fusionssignale detektiert und/oder analysiert werden und in einem nachfolgenden Schritt im Falle des Auftretens von Einzelsignalen eine Detektion und/oder Analyse der Mischsignale und deren Zuordnung zu den detektierten Chromosomen- und/oder DNA-Bereichen erfolgt.

Insbesondere kann dies erfolgen, indem zunächst bei der Analyse des Signalmusters ein Filter verwendet wird, durch welchen lediglich die durch die ersten Detektionslabel erzeugten Signale, d. h. die Fusionssignale bzw. potentiellen Einzelsignale, sichtbar sind. Da nur beim Auftreten von Einzelsignalen in dem Signalmuster Chromosomenaberrationen vorliegen, welche einer weiterführenden Analyse bedürfen, werden insbesondere durch Verwendung eines anderen Filters, insbesondere eines zur Darstellung der Mischsignale geeigneten Filters, auch die Mischsignale dargestellt, anhand derer - in Zusammenschau mit der Position der Fusions- bzw. Einzelsignale in dem Signalmuster - Chromosomenaberrationen einem spezifischen Chromosomen- bzw. DNA-Bereich zugeordnet werden können.

Zudem kann es gemäß einer besonderen Ausführungsform des erfindungsgemäßen Verfahrens vorgesehen sein, dass die Detektion des Signalmusters mittels computergestützter Analyse erfolgt. Dies ist insbesondere von Vorteil, wenn zur Erzeugung von Mischsignalen Hybridisierungssonden mit mehr als mindestens einem weiteren Detektionslabel, vorzugsweise mindestens zwei weiteren, vorzugsweise mehreren weiteren, Detektionslabeln in unterschiedlichen bzw. definierten Verhältnissen zueinander, markiert sind. Computergestützte Analysen erlauben anhand von Messungen der zugrundeliegenden Farbanteile bzw. Farben auch die Unterscheidung von Mischsignalen, welche mit bloßem Auge bzw. bei Betrachtung im Fluoreszenzmikroskop nicht voneinander unterscheidbar wären.

Erfindungsgemäß kann es somit insbesondere vorgesehen sein, dass der Nachweis von Translokationen bzw. Inversionen unter Verwendung von bis zu vierundzwanzig lokusspezifischen Hybridisierungssonden erfolgt, wobei jeweils zwei lokusspezifische Hybridisierungssonden distal und proximal einen jeweiligen Bruchpunktbereich flankieren und einzelne lokusspezifische Hybridisierungssonde dieser Sondenpaare gleichzeitig mit weiteren Detektionslabeln markiert sind. Vorzugsweise können somit können in einem Ansatz bis zu zwölf unterschiedliche Bruchpunktbereiche mit bis zu zwölf unterschiedlichen Break-Apart-Ansätzen zur Darstellung von bis zu zwölf Translokationen bzw. Inversionen untersucht werden. Der Nachweis einer spezifischen Translokation erfolgt über die Identifikation der Trennungen der Sondenpaare bzw. der Fusionssignale der Sondenpaare und anhand der jeweiligen Mischfarben bzw. Mischsignale.

Es ist ebenfalls möglich, mithilfe des erfindungsgemäßen Verfahrens Translokationen und Inversionen unter Verwendung von bis zu vierundzwanzig lokusspezifischen Hybridisierungssonden nachzuweisen, wobei jeweils zwei Sonden distal und proximal einen jeweiligen Bruchpunktbereich flankieren, diese Sondenpaare jeweils mit den gleichen Labeln A und B markiert sind, wobei jeweils eine Sonde mit Label A und die andere Sonde jeweils mit Label B markiert ist, und einzelne Sonden dieser Sondenpaare gleichzeitig mit weiteren Detektionslabeln X markiert sind. Der Nachweis einer spezifischen Translokation und/oder Inversion erfolgt über Veränderung spezifischer Fusions- und Mischsignale A-B/X bei einer Chromosomenaberration zu neuen und separaten Mischsignalen A/X und/oder B/X. Dabei kann wahlweise auch bei einem Sondenpaar kein weiteres Label X verwendet werden, so dass nur für dieses Sondenpaar die üblichen separaten Signal A und/oder B im Falle der zugrunde liegenden Aberration entstehen.

Somit ist es erstmals auch möglich, dass in einer ersten Analyse mehrerer unterschiedlicher potentiell nachweisbarer struktureller Chromosomenmutationen nur der Signale A und B unter Nutzung spezifischer Filtersysteme, z.B. Doppelfilter für die Signale A und B, die nur die Signale A und B, nicht aber weitere Label X sichtbar machen, zunächst schnell die Aussage erfolgen kann, ob überhaupt ein Break Apart der Fusionssignale A-B erfolgt ist und generell eine Translokation bzw. Inversion vorliegt. Nur bei positiven Auftreten separater Signale A bzw. B erfolgt dann die Auswertung der Mischsignale unter Beteiligung der Label X und damit die eindeutige Zuordnung der zugrundeliegenden Translokation.

Nachfolgend werden zudem weitere Besonderheiten bzw. Ausgestaltungsmöglichkeiten des erfindungsgemäßen Verfahrens beschrieben, welche für alle zuvor geschilderten möglichen Ausführungsformen des erfindungsgemäßen Verfahrens entsprechend gelten:
Was die einzelnen, durch jeweils eine einzelne lokusspezifische Hybridisierungssonde zu detektierenden Chromosomen- bzw. DNA-Bereiche anbelangt, so weisen diese im Rahmen der vorliegenden Erfindung vorzugsweise eine Länge von weniger als 5 Mbp, insbesondere weniger als 2 Mbp, vorzugsweise weniger als 1 Mbp, bevorzugt weniger als 750 kbp, besonders bevorzugt weniger als 500 kbp, auf. Gleichermaßen kann es vorgesehen sein, dass der durch eine einzelne lokusspezifische Hybridisierungssonde zu detektierende Chromosomen- und/oder DNA-Bereich von mindestens 500 bp, insbesondere mindestens 1 kbp, vorzugsweise mindestens 5 kbp, bevorzugt mindestens 10 kbp, aufweist. Schließlich kann es auch vorgesehen sein, dass der durch eine einzelne lokusspezifische Hybridisierungssonde zu detektierende Chromosomen- und/oder DNA-Bereich eine Länge im Bereich von 500 bp bis 5 Mbp, insbesondere im Bereich von 1 kbp bis 2 Mbp, vorzugsweise im Bereich von 5 kbp bis 1 Mbp, bevorzugt im Bereich von 10 kbp bis 750 kbp, besonders bevorzugt im Bereich von 10 kbp bis 500 kbp, aufweist.

Was darüber hinaus die weitere Ausgestaltung der erfindungsgemäß eingesetzten lokusspezifischen Hybridisierungssonden anbelangt, so liegen diese vorzugsweise in Form von Nukleinsäurefragmenten, insbesondere in Form von Polynukleotiden, modifizierten Polynukleotiden, modifizierten Nukleinsäurefragmenten, Oligonukleotiden und/oder Oligonukleotiden, vor. Was speziell die modifizierten Nukleinsäurefragmente anbelangt, so kann es sich insbesondere um *locked nucleic acids* (LNA) oder Peptid-Nukleinsäuren (PNA) handeln.

Gemäß einer ersten Ausführungsform der vorliegenden Erfindung kann es zudem vorgesehen sein, dass die lokusspezifischen Hybridisierungssonden durch jeweils ein einzelnes Nukleinsäurefragment gebildet werden, welches jeweils den zu detektierenden Chromosomen- und/oder DNA-Bereich abdeckt.

Gemäß einer weiteren und darüber hinaus bevorzugten Ausführungsform der vorliegenden Erfindung kann es auch vorgesehen sein, dass die lokusspezifischen. Hybridisierungssonden jeweils durch eine Vielzahl von Nukleinsäurefragmenten ("Sondenfragmenten") gebildet werden, welche jeweils den zu detektierenden Chromosomen- und/oder DNA-Bereich abdecken. Diesbezüglich ist es zudem bevorzugt, wenn die einzelnen Nukleinsäurefragmente ("Sondenfragmente") einer lokusspezifischen Hybridisierungssonde eine Länge im Bereich von 5 bis 2.000 bp, insbesondere im Bereich von 10 bis 1.500 bp, vorzugsweise im Bereich von 50 bis 1.000 bp, aufweisen

Was darüber hinaus die Erzeugung von Mischsignalen durch die Markierung von lokusspezifischen Hybridisierungssonden mit mindestens einem weiteren, von dem ersten verschiedenen Detektionslabel zur zielgerichteten Erzeugung von Mischsignalen anbelangt, so kann diese auf unterschiedliche Art und Weise erfolgen:
Gemäß einer diesbezüglich ersten Ausführungsform der vorliegenden Erfindung kann es vorgesehen sein, dass zur Erzeugung von Mischsignalen die Nukleinsäurefragmente ("Sondenfragmente") einer lokusspezifischen Hybridisierungssonde neben dem ersten Detektionslabel mit einem weiteren, von dem ersten Detektionslabel verschiedenen Detektionslabel markiert sind. Auf diese Art und Weise markierte Hybridisierungssonden erzeugen somit ein Mischsignal, welches lediglich auf zwei voneinander verschiedenen Detektionslabeln basiert.

Darüber hinaus kann es gemäß einer weiteren Ausführungsform der vorliegenden Erfindung - insbesondere vor dem Hintergrund, die Bandbreite an spezifischen Mischsignalen zu erhöhen bzw. die Zahl spezifischer bzw. voneinander unterscheidbarer Mischsignale zu erhöhen - vorgesehen sein, dass zur Erzeugung von Mischsignalen die Nukleinsäurefragmente ("Sondenfragmente") einer lokusspezifischen Hybridisierungssonde neben dem ersten Detektionslabel mit mehreren, insbesondere zwei bis zwanzig, vorzugsweise zwei bis zehn, bevorzugt zwei bis sechs, von dem ersten Detektionslabel verschiedenen Detektionslabeln markiert sind. Dabei kann es insbesondere vorgesehen sein, dass die Detektionslabel in voneinander verschiedenen Mengen eingesetzt sind.

Erfindungsgemäß kann es somit vorgesehen sein, dass zur Erzeugung von Mischsignalen im Rahmen der Markierung von lokusspezifischen Hybridisierungssonden mehrere voneinander verschiedene Detektionslabel in jeweils voneinander verschiedenen Verhältnissen eingesetzt werden. Beispielsweise - und keinesfalls beschränkend - kann eine lokusspezifische Hybridisierungssonde - neben dem ersten Detektionslabel - drei weitere Detektionslabel aufweisen, wobei dabei der Anteil des ersten Detektionslabels 20 %, der Anteil des zweiten Detektionslabels 60 % und der Anteil des dritten Detektionslabels 20 % beträgt, bezogen auf die drei weiteren Detektionslabel.

Gemäß dieser Ausführungsform kann es somit vorgesehen sein, dass die Hybridisierungssonden neben dem ersten Detektionslabel mindestens zwei, vorzugsweise mehrere voneinander verschiedene Detektionslabel aufweisen. Durch den Einsatz der verschiedenen Detektionslabel in voneinander verschiedenen Verhältnissen kann insgesamt die Zahl an spezifischen, insbesondere voneinander unterscheidbaren Mischsignalen erhöht werden, was wiederum den Nachweis einer größeren Zahl an detektierbaren Chromosmenaberrationen ermöglicht.

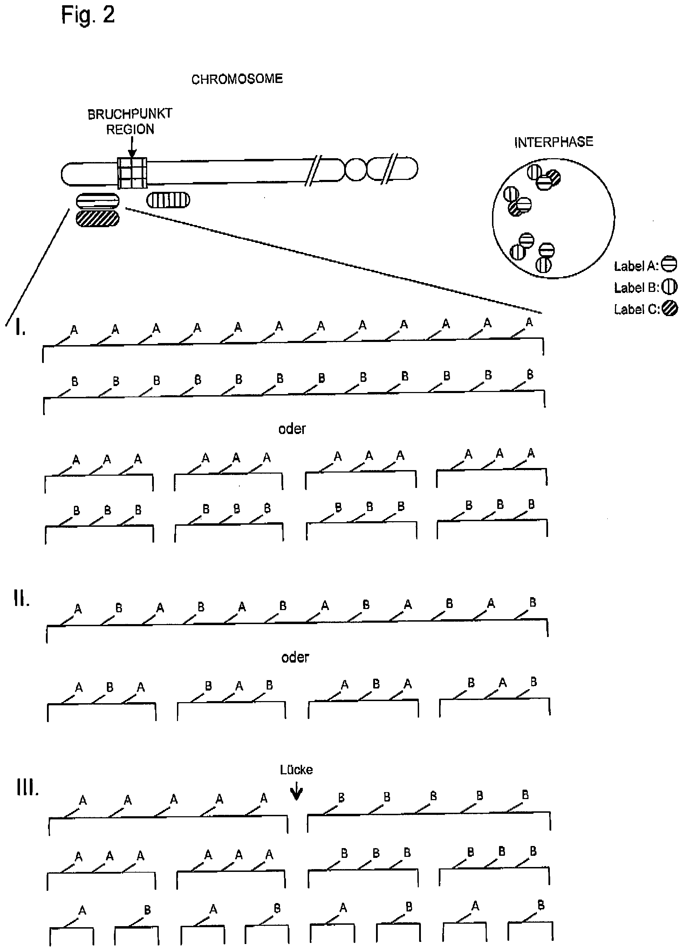

Im Rahmen der Markierung von lokusspezifischen Hybridisierungssonden kann es erfindungsgemäß weiterhin grundsätzlich vorgesehen sein, dass einzelne Nukleinsäurefragmente einer hybridisierungsspezifischen Sonde mit nur einem Detektionslabel markiert sind. Dabei kann es sich um das erste Detektionslabel und/oder um jedes weitere Detektionslabel handeln kann (vgl. Fig. 2 I)).

Anders ausgedrückt es erfindungsgemäß somit möglich, dass eine erste Portion der Nukleinsäurefragmente einer lokusspezifischen Hybridisierungssonde nur mit dem ersten Detektionslabel markiert ist und weitere Portionen der Nukleinsäurefragmente einer lokusspezifischen Hybridisierungssonde mit jeweils einem weiteren, von dem ersten Detektionslabel verschiedenen Detektionslabel markiert sind. Die Erzeugung von Mischsignalen kann somit gemäß einer Ausführungsform der vorliegenden Erfindung erfolgen, indem die das Mischsignal bildenden voneinander verschiedenen Detektionslabel auf voneinander verschiedenen Nukleinsäurefragmenten einer hybridisierungsspezifischen Sonde vorliegen (vgl. Fig. 2 I)).

Weiterhin kann es vorgesehen sein, dass einzelne Nukleinsäurefragmente einer hybridisierungsspezifischen Sonde mit mehreren, voneinander verschiedenen Detektionslabeln markiert sind, insbesondere wobei es sich dabei um das erste Detektionslabel und/oder um jedes weitere Detektionslabel handeln kann (vgl. Fig. 2 II)).

Gemäß dieser Ausführungsform der vorliegenden Erfindung kann es somit vorgesehen sein, dass die das Mischsignal bildenden, voneinander verschiedenen Detektionslabel auf gleichen Nukleinsäurefragmenten einer hybridisierungsspezifischen Sonde vorliegen bzw. gemeinsam auf den Nukleinsäurefragmenten einer hybridisierungsspezifischen Sonde vorliegen (sogenannte "Mischsonde") (vgl. Fig. 2 II)).

Darüber hinaus kann es im Rahmen auch vorgesehen, die beiden vorgenannten Ausführungsformen zur Erzeugung von Mischsignalen miteinander zu kombinieren, d.h. dass ein Teil der ein Mischsignal ausbildenden Nukleinsäurefragmente einer lokusspezifischen Hybridisierungssonde mit nur einem Detektionslabel markiert ist und ein weiterer oder mehrere weitere Teile der Nukleinsäurefragmente einer hybridisierungsspezifischen Sonde mit mindestens zwei verschiedenen Detektionslabeln markiert sind.

Darüber hinaus ist es auch möglich, dass Mischsignale erzeugt werden, wenn zwischen einzelnen hybridisierten Nukleinsäurefragmenten einer mit mindestens einem weiteren Detektionslabel markierten lokusspezifischen Hybridisierungssonde eine Beabstandung von maximal 3 Mbp, insbesondere maximal 2,5 Mbp, vorzugsweise maximal 2 Mbp, bevorzugt maximal 1 Mbp, besonders bevorzugt maximal 500 kb, noch mehr bevorzugt maximal 200 kb, vorliegt. Anders ausgedrückt ist es somit erfindungsgemäß auch möglich, ein Mischsignal zu erzeugen, wenn zwisehen den einzelnen hybridisierten Nukleinsäurefragmenten einer mit mindestens einem weiteren Detektionslabel markierten lokusspezifischen Hybridisierungssonde im hybridisierten Zustand eine "Lücke" von maximal 3 Mbp, insbesondere maximal 2,5 Mbp, vorzugsweise maximal 2 Mbp, bevorzugt maximal 1 Mbp, besonders bevorzugt maximal 500 kb, noch mehr bevorzugt maximal 200 kb, vorliegt (vgl. Fig. 2 III)).

Somit können Mischsignale insbesondere durch die Verwendung von "Mischlabeln" einer einzelnen Sonde erzeugt werden. Insbesondere können zur Entstehung der für eine chromosomale Region oder einen genomischen Abschnitt spezifischen Mischsignale wahlweise I) alle Fragmente einer Sonde oder wahlweise auch nur einzelne Fragmente einer Sonde mit mehreren Labeln markiert sein oder II) gleiche Fragmente mit jeweils unterschiedlichen Labeln markiert sein oder III) alternierend Fragmente mit unterschiedlichen Labeln markiert sein, so dass auch hier final nur ein Mischsignal sichtbar bzw. detektierbar ist (vgl. Fig. 2I bis III)).

Mischlabel und Mischsignale im Sinne des erfindungsmäßigen Verfahrens können dabei auch entstehen, wenn einzelne oder alle der unter I) bis I) vorgenannten Fragmente sich nur teilweise überlappen.

Mischlabel können auch im Sinne des erfindungsmäßigen Verfahrens entstehen, wenn einzelnen Fragmente oder Fragmentgruppen einer Sonde, die mit mindestens einem Label markiert sind, und andere einzelnen Fragmente oder Fragmentgruppen der Sonde, die mit mindestens einem weiteren Label markiert sind, einen Abstand von 2 Mbp, wahlweise 1 Mbp, wahlweise 500 kb und wahlweise 200 kb aufweisen.

Mischlabel und Mischsignale im Sinne des erfindungsmäßigen Verfahrens können somit auch entstehen, wenn zwei oder mehr sequenzgleiche oder nahezu sequenzgleiche Sonden verwendet werden, d.h. zwei oder mehrere Sonden die gleiche spezifische chromosomale Regionen oder gleiche genomische Abschnitte adressieren, aber mit unterschiedlichen Labeln markiert sind, wobei die genannten Sonden auch nur zu 95 %, wahlweise 90 %, wahlweise 80 %, wahlweise 70 %, wahlweise 60 %, wahlweise 50 % übereinstimmen können, wobei Unterschiede entweder durch Sequenzvariationen grundsätzlicher ähnlicher Sequenzen oder durch teilweise Überlappung nur einzelnen Bereiche der Sonden entstehen.

Die Auswahl von geeigneten Detektionslabeln als solche zur Durchführung liegt im üblichen Können des Fachmanns und erfolgt in Abhängigkeit von der eingesetzten Methode zur Durchführung der *In-Situ*-Hybridisierung. Üblicherweise kann durch die Auswahl geeigneter Detektionslabel eine direkte oder eine indirekte Markierung der Hybridisierungssonden erfolgen.

Besonders gute Ergebnisse werden im Rahmen der vorliegenden Erfindung erhalten, wenn die Detektionslabel ausgewählt sind aus der Gruppe von Farbstoffen; Farbstoffsubstraten; Chemielumineszenzfarbstoffen, insbesondere Acridinum; Radioisotopen; Spin-Labeln; Enzymen, insbesondere Alkalische Phosphatase, Meerrettich-Peroxidase, Sojabohnen-Peroxidase und/oder beta-Galactosidase; Haptenen, insbesondere Digoxigenin, Biotin, 2,4-Dinitrophenol, 5(6)-Carboxyfluorescein, Rhodamin, Bromdeoxyuridin, Acetylaminofluoren, Trinitrophenol, Trinitrophenol-Derivaten, Estradiol und/oder 2,4-Dinitrophenol; Quantum Dots; Beads; Aminohexylen; Pyrenen; und/oder Fluoreszenzfarbstoffen, insbesondere Fluorecein, Fluorescein-Derivat, 5(6)-Carboxyfluorescein, Coumarin, Coumarin-Derivat, Rhodamin, Rhodamin-Derivat, Tetramethylrhodamin, Lissamin, Texas Red, AMCA, TRITC, IR Farbstoff, Alexa-Farbstoff, Dyomics-Farbstoff, Phycoerythrine, Cascade Blue, Oregon Green 488, Pacific Blue und/oder Rhodamine Green.

Was die Durchführung der *In-Situ*-Hybridisierung als solche anbelangt, so kann diese auf verschiedene Art und Weise erfolgen.

Insbesondere kann es gemäß einer ersten bevorzugten Ausführungsform der *In-Situ*-Hybridisierung vorgesehen sein, dass diese unter direkter Markierung der Hybridisierungssonden, insbesondere mittels Fluoreszenz-*In-Situ*-Hybridisierung (FISH), erfolgt.

Gleichermaßen kann es vorgesehen sein, dass die *In-Situ*-Hybridisierung unter Markierung der Hybridisierungssonden mit Fluoreszenzfarbstoffen, insbesondere für den sichtbaren, infraroten und/oder ultravioletten Emissionsbereich, vorzugsweise für die Emissionsbereiche Grün, Orange/Rot, Rot, Gold und/oder Blau, erfolgt.

Gemäß einer weiteren bevorzugten Ausführungsform der *In-Situ*-Hybridisierung kann es gleichermaßen vorgesehen sein, dass diese unter indirekter Markierung der Hybridisierungssonden, insbesondere mittels Hellfeld-*In-Situ*-Hybridisierung (BrISH), erfolgt.

Zudem kann es erfindungsgemäß vorgesehen sein, dass die *In-Situ*-Hybridisierung unter Markierung der Hybridisierungssonden mit Haptenen, insbesondere Biotin, Digoxigenin und/oder DNP, und anschließender Detektion mittels Antikörper-gekoppelter alkalischer Phosphatase, Antikörper-gekoppelter Peroxidase und/oder Antikörper-gekoppelter beta-Galactosidase erfolgt.

Was die Analyse von fluoreszenzbasierten *In-Situ*-Hybridisierungen anbelangt, so erfolgt diese vorzugsweise unter Verwendung spezifischer Einzel- bzw. Mehrfach-Filtersets, welche insbesondere die zielgerichtete Darstellung von Fusions- und Mischsignalen erlauben.

Darüber hinaus kann es vorteilhaft sein, insbesondere bei der Erzeugung von Mischsignalen auf Basis von mehr als mindestens einem weiteren Detektionslabel, insbesondere bei Erzeugung von Mischlabeln auf Basis von mindestens zwei weiteren, vorzugsweise mehreren weiteren. Detektionslabeln in unterschiedlichen bzw. definierten Verhältnissen zueinander, eine Auswertung mittels computergestützter Analyse vorzunehmen. Darüber hinaus kann es auch vorgesehen sein, insbesondere durch computergestützte Analyse, überlagerte Bilder bereitzustellen, welche eine gemeinsame Darstellung der Signalmuster verschiedener Einzel- bzw. Mehrfach-Filtersets erlauben.

Grundsätzlich lässt sich mit dem erfindungsgemäßen eine Vielzahl verschiedener Arten von Chromosomenaberrationen detektieren. Insbesondere kann das erfindungsgemäße Verfahren zum Nachweis von Translokationen, Inversionen, segmentellen Duplikationen, Deletionen, Insertionen, Duplikationen, Aneuplodien und Amplifikationen, insbesondere Translokationen und/oder Inversionen, eingesetzt werden.

Im Rahmen des erfindungsgemäßen Verfahrens kann es dabei vorgesehen sein, dass die Chromosomenaberrationen im Zusammenhang mit Erkrankungen, insbesondere Malignomen, vorzugsweise Karzinomen, Sarkomen und/oder Leukämien, stehen.

Die auf potentielle Chromosomenaberrationen zu untersuchenden Gene sind vorzugsweise ausgewählt aus der Gruppe von ALK, ROS1, RET, NRG1, NTRK1, CARS, EML4, FGFR2, FGFR3, KIF5B, TGF, BCR, ABL, ALK, BCL2, BCL6, BIRC3, CCND1, EGR1, ETV6, FGFR1, FGFR3, IGH, KMT2A, MYC, PML, RARA, RUNX1, RUNX1T1, EWSR1, CHOP, FUS, COL1A1, DDIT3, JAZF1, NR4A3, FOXO1, FUS, PAX3, PAX7, PDGFB, SS18, TFE3, USP6, WT1, HER2/ERBB2, FGFR1, ALK, CCND1, CDK4, CD274, PDCD1LG2, EGR1, EGFR, ESR1, ETV1, FGF3,4,19, FGFR2, FGFR3, FHIT (RCC), KRAS, MDM2, MDM4, MET, MYB, MYC, MYCN, PIK3CA, PTEN, SMARCB1, SOX2, TERT, TOP2A, TP53, TYMS und/oder VHL.

Besonders gute Ergebnisse werden im Rahmen des erfindungsgemäßen Verfahrens erzielt, wenn das erfindungsgemäße Verfahren zur Detektion von Inversionen und/oder Translokationen eingesetzt wird:
Im Rahmen einer bevorzugten Ausführungsform der vorliegenden Erfindung wird das erfindungsgemäße Verfahren zum Nachweis unterschiedlicher Translokationen und/oder Inversionen, insbesondere in Lungentumoren, wobei insbesondere die Gene ALK, ROS1, RET, NRG1, NTRK1, CARS, EML4, FGFR2, FGFR3, KIF5B und/oder TGF betroffen sind, eingesetzt.

Weiterhin kann es vorgesehen sein, dass das erfindungsgemäße Verfahren zum Nachweis unterschiedlicher Translokationen und/oder Inversionen, insbesondere in Lymphomen und Leukämien, wobei insbesondere die Gene BCR, ABL, ALK, BCL2, BCL6, BIRC3, CCND1, EGR1, ETV6, FGFR1, FGFR3, IGH, KMT2A, MYC, PML, RARA, RUNX1 und /oder RUNX1T1 betroffen sind, eingesetzt wird.

Gemäß einer weiteren bevorzugten Ausführungsform der vorliegenden Erfindung wird das erfindungsgemäße Verfahren zum Nachweis unterschiedlicher Translokationen und/oder Inversionen, insbesondere in Sarkomen, eingesetzt, wobei insbesondere die Gene EWSR1, CHOP, FUS, COL1A1, DDIT3, JAZF1, NR4A3, FOXO1, FUS, PAX3, PAX7, PDGFB, SS18, TFE3, USP6 und/oder WT1 betroffen sind.

Auch kann es erfindungsgemäß vorgesehen sein, dass das erfindungsgemäße Verfahren zum Nachweis von Inversionen und/oder Translokationen eingesetzt wird, wobei insbesondere die Gene ALK und ROS1 betroffen sind.

Darüber hinaus werden im Rahmen der vorliegenden Erfindung hervorragende Ergebnisse erzielt, wenn das erfindungsgemäße Verfahren zur Detektion von Amplifikationen und/oder Deletionen eingesetzt wird:
Zum Nachweis Amplifikationen bzw. Deletionen können bis zu vierundzwanzig verschiedene lokusspezifische Sonden eingesetzt werden, wobei jeweils eine Sonde eine jeweilige genomische Region adressiert und wobei die verschiedene Sonden mit verschiedenen Labeln in unterschiedlichen Kombinationen und Verhältnissen markiert. Anhand der entstehenden Mischsignale in dem Signalmuster lassen sich die verschiedenen lokusspezifischen Sonden eindeutig voneinander unterscheiden. Somit können in einem Verfahren bis zu vierundzwanzig unterschiedliche Amplifikations- und/oder Deletionsereignisse der betroffenen genomischen Regionen untersucht werden. Der Nachweis einer spezifischen Amplifikation oder Deletion erfolgt über die Auszählung der verschiedenen Mischsignale bzw. Mischfarben.

Bevorzugt wird das erfindungsgemäße Verfahren zum Nachweis unterschiedlicher Amplifikationen und Deletionen, insbesondere in Brust-, Darm- und Lungentumoren eingesetzt, wobei insbesondere die Gene HER2/ERBB2, FGFR1, ALK, CCND1, CDK4 , CD274, PDCD1LG2, EGR1, EGFR, ESR1, ETV1, FGF3,4,19, FGFR2, FGFR3, FHIT (RCC), KRAS, MDM2, MDM4, MET, MYB, MYC, MYCN, PIK3CA, PTEN, SMARCB1, SOX2, TERT, TOP2A, TP53, TYMS und/oder VHL betroffen sind.

Insgesamt wurde somit im Rahmen der vorliegenden Erfindung überraschend gefunden, dass unter Verwendung lokusspezifischer Sonden mit Mischlabeln gut und sicher nachweisbare und auswertbare Mischsignale entstehen, die es erlauben, die von einer Aberration betroffenen chromosomalen Bereiche eindeutig zu identifizieren. Das erfindungsgemäße Verfahren erlaubt somit erstmals und überraschend den Nachweis mehrerer, verschiedener struktureller und/oder numerischer Chromosomenmutationen. Dies ist mit dem Stand der Technik nicht möglich.

Weiterer Gegenstand der vorliegenden Erfindung ist - gemäß einem **zweiten** erfindungsgemäßen Aspekt - eine Zusammensetzung zum Nachweis von mindestens zwei voneinander verschiedenen Chromosomenaberrationen, insbesondere strukturellen und/oder numerischen Chromosomenaberrationen, vorzugsweise strukturellen Chromosomenaberrationen, mittels *In-Situ*-Hybridisierung, insbesondere durch Detektion von Chromosomen- und/oder DNA-Bereichen in einer biologischen Probe, vorzugsweise in einer oder mehreren Zelle(n) und/oder in einem oder mehreren Zellkern(en), insbesondere mittels eines Verfahrens nach einem der vorangehenden Ansprüche, wobei die Zusammensetzung mindestens drei, vorzugsweise mindestens vier voneinander verschiedene, mit jeweils einem ersten Detektionslabel markierte, lokusspezifische Hybridisierungssonden umfasst, und wobei mindestens eine der
lokusspezifischen Hybridisierungssonden mit mindestens einem weiteren, von dem ersten verschiedenen Detektionslabel, bezogen auf die jeweilige lokusspezifische Hybridisierungssonde, markiert ist.

Gleichermaßen ist Gegenstand der vorliegenden Erfindung - gemäß diesem erfindungsgemäßen Aspekt - eine Zusammensetzung zur Verwendung bei der prophylaktischen und/oder therapeutischen Behandlung und/oder bei der Diagnose und/oder Prognose von im Zusammenhang mit Chromosomenaberrationen stehenden Erkrankungen, insbesondere Malignomen, vorzugsweise Karzinomen, Sarkomen und/oder Leukämien, besonders bevorzugt Lungentumoren, Lymphomen, Leukämien, Sarkomen, Mammakarzinomen und/oder Darmkrebs, wobei die Zusammensetzung mindestens drei, vorzugsweise mindestens vier voneinander verschiedene, mit jeweils einem ersten Detektionslabel markierte, lokusspezifische Hybridisierungssonden umfasst, und wobei mindestens eine der lokusspezifischen Hybridisierungssonden mit mindestens einem weiteren, von dem ersten verschiedenen Detektionslabel, bezogen auf die jeweilige lokusspezifische Hybridisierungssonde, markiert ist.

In diesem Zusammenhang kann es insbesondere vorgesehen sein, dass die erfindungsgemäßen Zusammensetzungen zur Durchführung eines Verfahrens, wie es zuvor beschrieben wurde, bestimmt sind bzw. eingesetzt werden.

In Bezug auf weitergehende Einzelheiten zu diesem Erfindungsaspekt kann auf die vorstehenden Ausführungen zu den übrigen erfindungsgemäßen Aspekten verwiesen werden, welche entsprechend auch in Bezug auf diesen Erfindungsaspekt gelten.

Darüber hinaus ist Gegenstand der vorliegenden Erfindung - gemäß einem **dritten** erfindungsgemäßen Aspekt - die Verwendung einer Zusammensetzung, insbesondere wie sie zuvor beschrieben wurde, zum Nachweis von mindestens zwei voneinander verschiedenen Chromosomenaberrationen, insbesondere strukturellen und/oder numerischen Chromosomenaberrationen, vorzugsweise strukturellen Chromosomenaberrationen, mittels *In-Situ-*Hybridisierung, insbesondere durch Detektion von Chromosomen- und/oder DNA-Bereichen in einer biologischen Probe, vorzugsweise in einer oder mehreren Zelle(n) und/oder in einem oder mehreren Zellkern(en), insbesondere mittels des zuvor beschriebenen Verfahrens.

In Bezug auf weitergehende Einzelheiten zu diesem Erfindungsaspekt kann auf die vorstehenden Ausführungen zu den übrigen erfindungsgemäßen Aspekten verwiesen werden, welche entsprechend auch in Bezug auf diesen Erfindungsaspekt gelten.

Weiterer Gegenstand der vorliegenden Erfindung ist zudem - gemäß einem **vierten** erfindungsgemäßen Aspekt - die Verwendung von mindestens drei, vorzugsweise mindestens vier voneinander verschiedenen, mit jeweils einem ersten Detektionslabel markierten, lokusspezifischen Hybridisierungssonden, wobei mindestens eine der lokusspezifischen Hybridisierungssonden mit mindestens einem weiteren, von dem ersten verschiedenen Detektionslabel, bezogen auf die jeweilige lokusspezifische Hybridisierungssonde, markiert ist, zum Nachweis von Chromosomenaberrationen, insbesondere strukturellen und/oder numerischen Chromosomenaberrationen, mittels *In-Situ*-Hybridisierung, insbesondere durch Detektion von Chromosomen- und/oder DNA-Bereichen in einer biologischen Probe, vorzugsweise in einer oder mehreren Zelle(n) und/oder in einem oder mehreren Zellkern(en), vorzugsweise mittels eines Verfahrens, wie es zuvor beschrieben wurde.

Gleichermaßen ist Gegenstand der vorliegenden Erfindung gemäß diesem Erfindungsaspekt die Verwendung von mindestens drei, vorzugsweise mindestens vier voneinander verschiedenen, mit jeweils einem ersten Detektionslabel markierten, lokusspezifischen Hybridisierungssonden, wobei mindestens eine der lokusspezifischen Hybridisierungssonden mit mindestens einem weiteren, von dem ersten verschiedenen Detektionslabel, bezogen auf die jeweilige lokusspezifische Hybridisierungssonde, markiert ist, vorzugsweise im Rahmen eines zuvor beschriebenen erfindungsgemäßen Verfahrens, bei der Diagnose und/oder Prognose von im Zusammenhang mit Chromosomenaberrationen stehenden Erkrankungen, insbesondere Malignomen, vorzugsweise Karzinomen, Sarkomen und/oder Leukämien, besonders bevorzugt Lungentumoren, Lymphomen, Leukämien, Sarkomen, Mammakarzinomen und/oder Darmkrebs.

In Bezug auf weitergehende Einzelheiten zu diesem Erfindungsaspekt kann auf die vorstehenden Ausführungen zu den übrigen erfindungsgemäßen Aspekten verwiesen werden, welche entsprechend auch in Bezug auf diesen Erfindungsaspekt gelten.

Auch ist Gegenstand der vorliegenden Erfindung zudem - gemäß einem **fünften** erfindungsgemäßen Aspekt - die Verwendung von mindestens einer mit mindestens zwei Detektionslabeln markierten lokusspezifischen Hybridisierungssonde zusammen mit mindestens einer, insbesondere mindestens zwei, vorzugsweise mindestens drei weiteren, jeweils mit mindestens einem ersten Detektionslabel markierten, voneinander verschiedenen lokusspezifischen Hybridisierungssonden zum Nachweis von mindestens zwei voneinander verschiedenen Chromosomenaberrationen, insbesondere strukturellen und/oder numerischen Chromosomenaberrationen, vorzugsweise strukturellen Chromosomenaberrationen, mittels *In-Situ-*Hybridisierung, insbesondere durch Detektion von Chromosomen- und/oder DNA-Bereichen in einer biologischen Probe, vorzugsweise in einer oder mehreren Zelle(n) und/oder in einem oder mehreren Zellkern(en), vorzugsweise mittels eines Verfahrens, wie es zuvor beschrieben wurde.

In Bezug auf weitergehende Einzelheiten zu diesem Erfindungsaspekt kann auf die vorstehenden Ausführungen zu den übrigen erfindungsgemäßen Aspekten verwiesen werden, welche entsprechend auch in Bezug auf diesen Erfindungsaspekt gelten.

Schließlich ist Gegenstand der vorliegenden Erfindung - gemäß einem **sechsten** erfindungsgemäßen Aspekt - ein Kit bzw. Kit-of-parts oder Set zum Nachweis von mindestens zwei voneinander verschiedenen Chromosomenaberrationen, insbesondere strukturellen und/oder numerischen Chromosomenaberrationen, vorzugsweise strukturellen Chromosomenaberrationen, mittels *In-Situ*-Hybridisierung, insbesondere durch Detektion von Chromosomen- und/oder DNA-Bereichen in einer biologischen Probe, vorzugsweise in einer oder mehreren Zelle(n) und/oder in einem oder mehreren Zellkern(en), umfassend mindestens drei, vorzugsweise mindestens vier voneinander verschiedene, mit jeweils einem ersten Detektionslabel markierte, lokusspezifische Hybridisierungssonden, wobei mindestens eine der lokusspezifischen Hybridisierungssonden mit mindestens einem weiteren, von dem ersten verschiedenen Detektionslabel, bezogen auf die jeweilige Hybridisierungssonde, markiert ist, insbesondere wobei das Kit zur Durchführung des zuvor geschilderten Verfahrens bestimmt ist und/oder eingesetzt wird.

In diesem Zusammenhang kann es insbesondere vorgesehen sein, dass die mindestens drei, vorzugsweise mindestens vier voneinander verschiedenen lokusspezifischen Hybridisierungssonden in einer gemeinsamen Zusammensetzung, insbesondere in einer Zusammensetzung wie sie zuvor geschildert wurde, vorliegen. Gleichermaßen kann es vorgesehen sein, dass die mindestens drei, vorzugsweise mindestens vier voneinander verschiedenen lokusspezifischen Hybridisierungssonden getrennt voneinander in separaten Zusammensetzungen vorliegen.

In Bezug auf weitergehende Einzelheiten zu diesem Erfindungsaspekt kann auf die vorstehenden Ausführungen zu den übrigen erfindungsgemäßen Aspekten verwiesen werden, welche entsprechend auch in Bezug auf diesen Erfindungsaspekt gelten.

Nachfolgend wird die vorliegende Erfindung anhand von Zeichnungen und Beispielen näher beschrieben. Es zeigt:
- Fig. 1:: Schematische Darstellung eines erfindungsgemäßen Verfahrens für die Detektion von zwei Translokationen.
- Fig. 2:: Schematische Darstellung eines erfindungsgemäßen Verfahrens betreffend die Verwendung von mehreren Labeln zur Darstellung von Mischlabeln und Mischsignalen.
- Fig. 3:: Schema zu Signalmustern bei Verwendung einer Vierfach-FISH-Sonde "ZytoLight SPEC ALK & ROS1 Break Apart Dual-Mix NG-FISH Probe" der Fa. ZytoVision GmbH.
- Fig. 4:: Schema zu Signalmustern bei Verwendung einer Vierfach-FISH-Sonde "ZytoLight SPEC ALK & ROS1 Break Apart Single-Mix NG-FISH Probe" der Fa. ZytoVision GmbH.
- Fig. 5:: Schema zu Signalmustern bei Verwendung einer Sechsfach-FISH-Sonde "ZytoLight SPEC ALK & ROS1 & RET Break Apart Single-Mix NG-FISH Probe" der Fa. ZytoVision GmbH.
- Fig. 6:: Schema zu Signalmustern bei Verwendung einer Sechsfach-FISH-Sonde "ZytoLight SPEC ALK & ROS1 & RET Break Apart Single-Mix II NG-FISH Probe" der Fa. ZytoVision GmbH.
- Fig. 7:: Schematische Darstellung eines erfindungsgemäßen Verfahrens für die Detektion von vier numerischen Aberrationen

Fig. 1 zeigt eine schematische Darstellung eines erfindungsgemäßen Verfahrens für die Detektion von zwei Translokationen unter Verwendung von vier Sonden und drei Labeln, wobei eine Sonde gleichzeitig mit zwei Labeln markiert ist. Es zeigt das Signalmuster bei normalen Zellen sowie bei Zellen mit Translokation des ALK-Gens in 2p23 oder des ROS1-Gens in 6q22. Beide Bruchpunktregionen (ALK und ROS1) werden jeweils von Label A bzw. B der Vierfach-ISH-Sonde flankiert und ergeben jeweils ein Fusionssignal A-B. Eine Seite der ALK Bruchpunktregion wird darüber hinaus auch mit Label C flankiert, so dass es zu einem Mischlabel A/C kommt.

In der Interphase einer normalen Zelle (ohne ALK oder ROS1 Aberrationen) werden die ROS1-Gen-Loki durch Fusionssignale A-B markiert und ALK-Gen-Loki durch Fusionssignale A-B, die mit A/C Mischsignalen einhergehen, markiert. In der Interphase einer von einer ALK-Translokation betroffenen Zelle wird das durch die Translokation betroffene ALK-Gen durch ein separates Signal des Labels B sowie ein davon separates Mischsignal A/C markiert. In der Interphase einer von einer ROS1-Translokation betroffenen Zelle wird das durch die Translokation betroffene ROS1-Gen durch ein separates Signal des Labels A sowie ein separates Signal des Labels B markiert.

Fig. 2 zeigt eine schematische Darstellung eines erfindungsgemäßen Verfahrens betreffend die Verwendung von mehreren Labeln zur Darstellung von Mischlabeln und Mischsignalen. Der Übersicht halber werden nur zwei Label aufgeführt. Mischsignale, die spezifisch für eine lokusspezifische Sonden und somit für eine chromosomale Region oder einen genomischen Abschnitt sind, können entstehen, wenn I) Fragmente einer Sonde mit jeweils unterschiedlichen Labeln markiert sind und/oder II) alle Fragmente einer Sonde oder wahlweise auch nur einzelne Fragmente einer Sonde mit mehreren Labeln markiert sind und/oder III) alternierend Fragmente mit unterschiedlichen Labeln markiert sein, so dass auch hier final nur ein Mischsignal sichtbar bzw. detektierbar ist. Dabei können sich alle oder auch nur einzelne Fragmente nach I) bis III) überlagern bzw. überlappen (nicht dargestellt) und es können auch Mischlabel entstehen, wenn einzelne Fragmente oder Fragmentgruppen nach I) bis III) einen Abstand von bis zu 2 Mbp, z.B. in der dargestellten "Lücke", aufweisen.

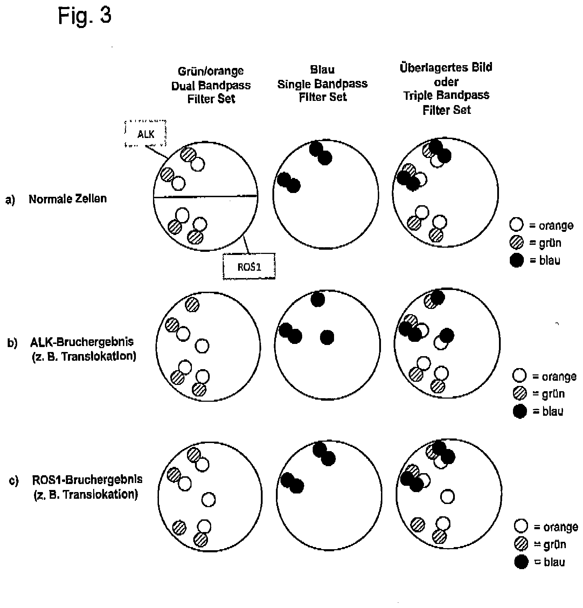

Fig. 3 zeigt ein Schema zu Signalmustern bei Verwendung einer entsprechenden Vierfach-FISH-Sonde "ZytoLight SPEC ALK & ROS1 Break Apart Dual-Mix NG-FISH Probe" der Fa. ZytoVision. Die Sonde besteht aus grün-markierten Polynukleotiden (Absorption bei 503 nm und Emission bei 528 nm), die in 2p23 gegen proximal zur ALK-Bruchpunktregion gelegene Sequenzen und in 6q22 gegen proximal zur ROS1-Bruchpunktregion gelegene Sequenzen gerichtet sind, orangemarkierten Polynukleotiden (Absorption bei 547 nm und Emission bei 572 nm), die in 2p23 gegen distal zur ALK-Bruchpunktregion gelegene Sequenzen und in 6q22 gegen distal zur ROS1-Bruchpunktregion gelegene Sequenzen gerichtet sind, sowie blau-markierten Polynukleotiden (Absorption bei 426 nm und Emission bei 480 nm), die in der Region 2p23 gegen distal und gegen proximal zur ALK-Bruchpunktregion gelegene Sequenzen gerichtet sind.

Bei Verwendung geeigneter Filtersätze erscheinen die Hybridisierungssignale für das nicht-rearrangierte ALK-Gen als grün-orange Fluoreszenz-Fusionssignale, die sich aus grün/blauen und orange/blauen Fluoreszenz-Mischsignalen zusammensetzen. Die Hybridisierungssignale für das nicht-rearrangierte ROS1-Gen erscheinen als grün-orange Fluoreszenz-Fusionssignale.

In der Interphase einer normalen Zelle (ohne ALK oder ROS1 Aberrationen) erscheinen vier grün-orange Fusionssignale bei Verwendung eines geeigneten grün-orange Dual-Bandpass-Filtersets, zwei blaue Signale bei Verwendung eines geeigneten Single-Bandpass-Filtersets sowie bei Verwendung eines geeigneten Triple-Bandpass-Filtersets zwei grün-orange Fusionssignale und zwei grün-orange/blaue Fusions-und Mischsignale (vgl. Fig. 3a).

Ein von einer ALK-Translokation betroffener 2p23-Lokus ist durch ein separates grün/blaues Mischsignal und ein separates orange/blaues Mischsignal gekennzeichnet. (vgl. Fig. 3b).

Ein von einer ROS1-Translokation betroffener 6q22-Lokus ist durch ein separates grünes und ein separates oranges Signal gekennzeichnet (vgl. Fig. 3c).

Bei der Verwendung geeigneter Dual-Bandpass-Filtersets für grüne und orangene Signale lassen grüne und davon separate orange Signale somit zunächst nur die Aussage zu, dass grundsätzlich eine ALK- oder ROS1-Translokation vorliegt. Eine diagnostisch möglicherweise relevante Unterscheidung zwischen ALK- oder ROS1-Translokation kann dann unter Einbeziehung der blauen Fluoreszenzsignale erfolgen. Überlappen die separaten grünen Signale blaue Signale (grün/blaue Mischsignale) bzw. überlappen die separaten orangen Signale blaue Signale (orange/blaue Mischsignale), indiziert dies eine ALK-Translokation. Überlappen die separaten grünen und orangen Signale sich nicht mit blauen Signalen, indiziert dies eine ROS1-Translokation.

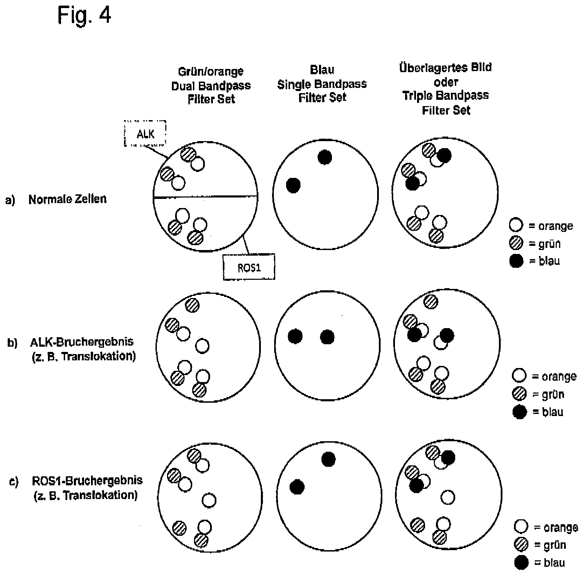

Fig. 4 zeigt ein Schema zu Signalmustern bei Verwendung einer entsprechenden Vierfach-FISH-Sonde "ZytoLight SPEC ALK & ROS1 Break Apart Single-Mix NG-FISH Probe" der Fa. ZytoVision. Die Sonde besteht aus grün-markierten Polynukleotiden (Absorption bei 503 nm und Emission bei 528 nm), die in 2p23 gegen proximal zur ALK-Bruchpunktregion gelegene Sequenzen und in 6q22 gegen proximal zur ROS1-Bruchpunktregion gelegene Sequenzen gerichtet sind, orangemarkierten Polynukleotiden (Absorption bei 547 nm und Emission bei 572 nm), die in 2p23 gegen distal zur ALK-Bruchpunktregion gelegene Sequenzen und in 6q22 gegen distal zur ROS1-Bruchpunktregion gelegene Sequenzen gerichtet sind, sowie blau-markierten Polynukleotiden (Absorption bei 426 nm und Emission bei 480 nm), die in der Region 2p23 gegen distal zur ALK-Bruchpunktregion gelegene Sequenzen gerichtet sind.

Bei Verwendung geeigneter Filtersätze erscheinen die Hybridisierungssignale für das nicht-rearrangierte ALK-Gen als grün-orange Fluoreszenz-Fusionssignale, die sich aus grünen und orange/blauen Fluoreszenz-Mischsignalen zusammensetzen. Die Hybridisierungssignale für das nicht-rearrangierte ROS1-Gen erscheinen als grün-orange Fluoreszenz-Fusionssignale.

In der Interphase einer normalen Zelle (ohne ALK oder ROS1 Aberrationen) erscheinen vier grün-orange Fusionssignale bei Verwendung eines geeigneten grün-orange Dual-Bandpass-Filtersets, zwei blaue Signale bei Verwendung eines geeigneten Single-Bandpass-Filtersets sowie bei Verwendung eines geeigneten Triple-Bandpass-Filtersets zwei grün-orange Fusionssignale und zwei grün-orange/blaue Fusions-und Mischsignale (vgl. Fig. 4a).

Ein von einer ALK-Translokation betroffener 2p23-Lokus ist durch ein separates grünes Signal und ein separates orange/blaues Mischsignal gekennzeichnet. (vgl. Fig. 4b).

Ein von einer ROS1-Translokation betroffener 6q22-Lokus ist durch ein separates grünes und ein separates oranges Signal gekennzeichnet (vgl. Fig. 4c).

Bei der Verwendung geeigneter Dual-Bandpass-Filtersets für grüne und orangene Signale lassen grüne und davon separate orange Signale somit zunächst nur die Aussage zu, dass grundsätzlich eine ALK- oder ROS1-Translokation vorliegt. Eine diagnostisch möglicherweise relevante Unterscheidung zwischen ALK- oder ROS1-Translokation kann dann unter Einbeziehung der blauen Fluoreszenzsignale erfolgen. Überlappen die separaten orangen Signale blaue Signale (orangelblaue Mischsignale), indiziert dies eine ALK-Translokation. Überlappen die separaten orangen Signale sich nicht mit blauen Signalen, indiziert dies eine ROS1- Translokation.

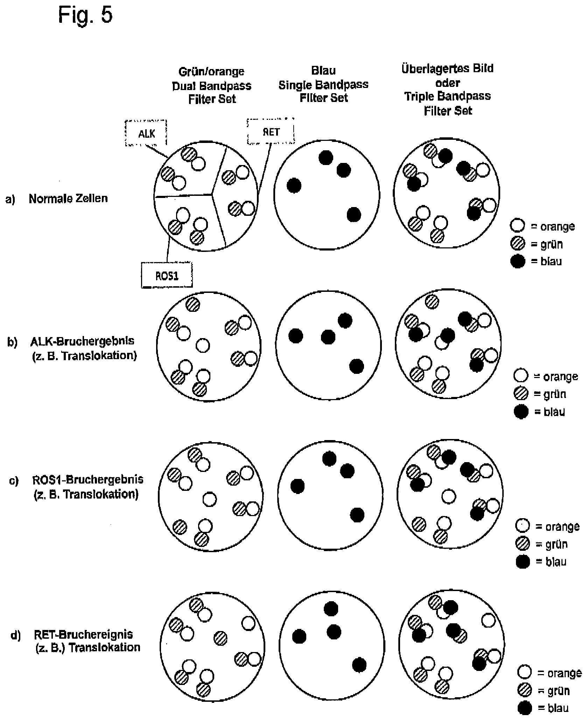

Fig. 5 zeigt ein Schema zu Signalmustern bei Verwendung einer entsprechenden Sechsfach-FISH-Sonde "ZytoLight SPEC ALK & ROS1 & RET Break Apart Dual-Mix NG-FISH Probe" der Fa. ZytoVision. Die Sonde besteht aus grün-markierten Polynukleotiden (Absorption bei 503 nm und Emission bei 528 nm), die in 2p23 gegen proximal zur ALK-Bruchpunktregion gelegene Sequenzen, in 6q22 gegen proximal zur ROS1-Bruchpunktregion gelegene Sequenzen und in 10q11 gegen proximal zur RET-Bruchpunktregion gelegene Sequenzen gerichtet sind, orangemarkierten Polynukleotiden (Absorption bei 547 nm und Emission bei 572 nm), die in 2p23 gegen distal zur ALK-Bruchpunktregion gelegene Sequenzen, in 6q22 gegen distal zur ROS1-Bruchpunktregion gelegene Sequenzen und in 10q11 gegen distal zur RET-Bruchpunktregion gelegene Sequenzen gerichtet sind sowie blau-markierten Polynukleotiden (Absorption bei 426 nm und Emission bei 480 nm), die in der Region 2p23 gegen distal zur ALK-Bruchpunktregion gelegene Sequenzen und in 10q11 gegen proximal zur RET-Bruchpunktregion gelegene Sequenzen gerichtet sind.

Bei Verwendung geeigneter Filtersätze erscheinen die Hybridisierungssignale für das nicht-rearrangierte ALK-Gen als grün-orange Fluoreszenz-Fusionssignale, die sich aus grünen und orange/blauen Fluoreszenz-Mischsignalen zusammensetzen. Die Hybridisierungssignale für das nicht-rearrangierte RET-Gen erscheinen als grün-orange Fluoreszenz-Fusionssignale, die sich aus grün/blauen Mischsignalen und orangen Signalen zusammensetzen. Die Hybridisierungssignale für das nicht-rearrangierte ROS1-Gen erscheinen als grün-orange Fluoreszenz-Fusionssignale.

In der Interphase einer normalen Zelle (ohne ALK, ROS1 oder RET Aberrationen) erscheinen sechs grün-orange Fusionssignale bei Verwendung eines geeigneten grün-orange Dual-Bandpass-Filtersets, vier blaue Signale bei Verwendung eines geeigneten Single-Bandpass-Filtersets sowie bei Verwendung eines geeigneten Triple-Bandpass-Filtersets zwei grün-orange Fusionssignale, zwei grünorangelblaue Fusions-und Mischsignale und zwei grün/blaue-orange Fusions-und Mischsignale (vgl. Fig. 5a).

Ein von einer ALK-Translokation betroffener 2p23-Lokus ist durch ein separates grünes Signal und ein separates orange/blaues Mischsignal gekennzeichnet. (vgl. Fig. 5b).

Ein von einer ROS1-Translokation betroffener 6q22-Lokus ist durch ein separates grünes und ein separates oranges Signal gekennzeichnet (vgl. Fig. 5c).

Ein von einer RET-Translokation betroffener 10q11-Lokus ist durch ein separates oranges Signal und ein separates grün/blaues Mischsignal gekennzeichnet. (vgl. Fig. 5d).

Bei der Verwendung geeigneter Dual-Bandpass-Filtersets für grüne und orangene Signale lassen grüne und davon separate orange Signale somit zunächst nur die Aussage zu, dass grundsätzlich eine ALK-, ROS1- oder RET-Translokation vorliegt. Eine diagnostisch möglicherweise relevante Unterscheidung zwischen ALK-, ROS1- oder RET-Translokation kann dann unter Einbeziehung der blauen Fluoreszenzsignale erfolgen. Überlappen die separaten orangen Signale blaue Signale (orange/blaue Mischsignale), indiziert dies eine ALK-Translokation. Überlappen die separaten grünen Signale blaue Signale (grün/blaue Mischsignale), indiziert dies eine RET-Translokation. Überlappen weder die separaten orangen noch die separaten grünen Signale sich mit blauen Signalen, indiziert dies eine ROS1- Translokation.

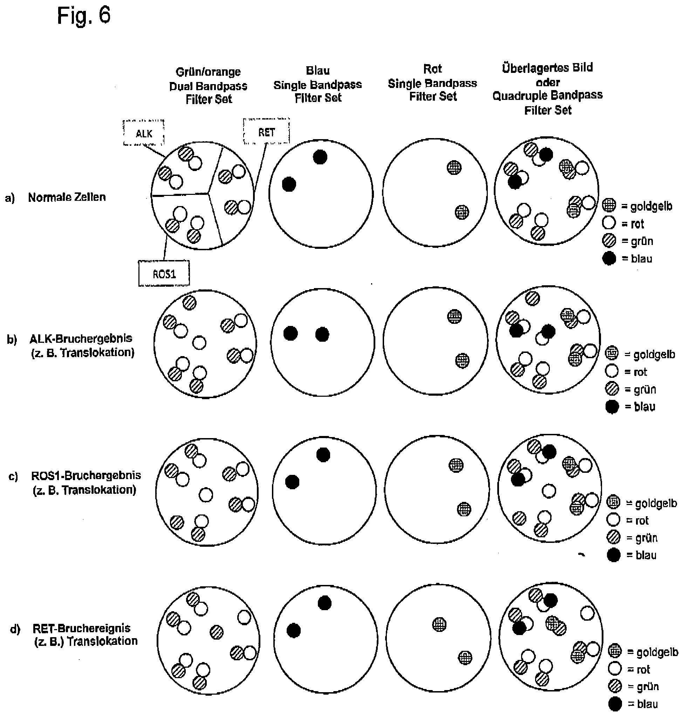

Fig. 6 zeigt ein Schema zu Signalmustern bei Verwendung einer entsprechenden Sechsfach-FISH-Sonde "ZytoLight SPEC ALK & ROS1 & RET Break Apart Dual-Mix II NG-FISH Probe" der Fa. ZytoVision GmbH. Die Sonde besteht aus grün-markierten Polynukleotiden (Absorption bei 503 nm und Emission bei 528 nm), die in 2p23 gegen proximal zur ALK-Bruchpunktregion gelegene Sequenzen, in 6q22 gegen proximal zur ROS1-Bruchpunktregion gelegene Sequenzen und in 10q11 gegen proximal zur RET-Bruchpunktregion gelegene Sequenzen gerichtet sind, rot-markierten Polynukleotiden (Absorption bei 580 nm und Emission bei 599 nm), die in 2p23 gegen distal zur ALK-Bruchpunktregion gelegene Sequenzen, in 6q22 gegen distal zur ROS1-Bruchpunktregion gelegene Sequenzen und in 10q11 gegen distal zur RET-Bruchpunktregion gelegene Sequenzen gerichtet sind, blau-markierten Polynukleotiden (Absorption bei 426 nm und Emission bei 480 nm), die in der Region 2p23 gegen distal zur ALK-Bruchpunktregion gelegene Sequenzen gerichtet sind sowie goldgelb-markierten Polynukleotiden (Absorption bei 532 nm und Emission bei 553 nm), die in der Region 10q11 gegen proximal zur RET-Bruchpunktregion gelegene Sequenzen gerichtet sind.

Bei Verwendung geeigneter Filtersätze erscheinen die Hybridisierungssignale für das nicht-rearrangierte ALK-Gen als grün-rote Fluoreszenz-Fusionssignale, die sich aus grünen und rot/blauen Fluoreszenz-Mischsignalen zusammensetzen. Die Hybridisierungssignale für das nicht-rearrangierte RET-Gen erscheinen als grün-rote Fluoreszenz-Fusionssignale, die sich aus grün/goldgelben Mischsignalen und roten Signalen zusammensetzen. Die Hybridisierungssignale für das nicht-rearrangierte ROS1-Gen erscheinen als grün-rote Fluoreszenz-Fusionssignale.

In der Interphase einer normalen Zelle (ohne ALK, ROS1 oder RET Aberrationen) erscheinen sechs grün-rote Fusionssignale bei Verwendung eines geeigneten grün-rot Dual-Bandpass-Filtersets, zwei blaue Signale bei Verwendung eines geeigneten Single-Bandpass-Filtersets sowie zwei goldgelbe Signale bei Verwendung eines geeigneten Single-Bandpass-Filtersets. (vgl. Fig. 6a).

Ein von einer ALK-Translokation betroffener 2p23-Lokus ist durch ein separates grünes Signal und ein separates rot/blaues Mischsignal gekennzeichnet. (vgl. Fig. 6b).

Ein von einer ROS1-Translokation betroffener 6q22-Lokus ist durch ein separates grünes und ein separates rotes Signal gekennzeichnet (vgl. Fig. 6c).

Ein von einer RET-Translokation betroffener 10q11-Lokus ist durch ein separates rotes Signal und ein separates grün/goldgelbes Mischsignal gekennzeichnet. (vgl. Fig. 6d).

Bei der Verwendung geeigneter Dual-Bandpass-Filtersets für grüne und rote Signale lassen grüne und davon separate rote Signale somit zunächst nur die Aussage zu, dass grundsätzlich eine ALK-, ROS1- oder RET-Translokation vorliegt. Eine diagnostisch möglicherweise relevante Unterscheidung zwischen ALK-, ROS1- oder RET-Translokation kann dann unter Einbeziehung der blauen bzw. goldgelben Fluoreszenzsignale erfolgen. Überlappen die separaten roten Signale blaue Signale (rot/blaue Mischsignale), indiziert dies eine ALK-Translokation. Überlappen die separaten grünen Signale goldgelbe Signale (grün/goldgelbe Mischsignale), indiziert dies eine RET-Translokation. Überlappen weder die separaten roten noch die separaten grünen Signale sich mit blauen bzw. goldgelben Signalen, indiziert dies eine ROS1-Translokation.

Fig. 7 zeigt eine schematische Darstellung eines erfindungsgemäßen Verfahrens für die Detektion von vier numerischen Aberrationen unter Verwendung von vier Sonden und vier Labeln, wobei drei Sonden gleichzeitig mit jeweils zwei Labeln markiert sind, wobei sich die für die Kombination verwendeten Label bei diesen drei Sonden voneinander unterscheidet. Es zeigt das Signalmuster bei normalen Zellen sowie bei Zellen mit Amplifikation des MET-Gens in 7q31. Die Region 17q11.2-q12 des ERBB2-Gens wird mit Label A überdeckt, die Region 7p12 des EGFR-Gens mit Label A und darüber hinaus Label D, so dass es zu einem Mischlabel A/D kommt, die Region 8p11.23-p11.22 des FGFR1-Gens mit Label A und darüber hinaus Label C, so dass es zu einem Mischlabel A/C kommt sowie die Region 7q31 des MET-Gens mit Label A und darüber hinaus Label B, so dass es zu einem Mischlabel A/B kommt.

In der Interphase einer normalen Zelle (ohne numerische ERBB2, EGFR, FGFR1 oder MET Aberration) werden sämtliche Loki durch Signale des Label A markiert. Eine Ko-Lokalisation eines Signals des Label A mit einem Signal des Label B führt zu einem Mischlabel A/B und markiert den MET-Gen-Lokus. Dem folgend markiert das Mischlabel A/C den FGFR1-Gen-Lokus und das Mischlabel A/D den EGFR-Gen-Lokus. Der ERBB2-Gen-Lokus zeichnet sich dadurch aus, dass es zu keiner Ko-Lokalisation mit einem anderen Label kommt. In der Interphase einer Zelle mit MET-Gen-Amplifikation kommt es zu einer Zunahme von Signalen des Labels A, welche mit Signalen des Labels B ko-lokalisieren, somit zu einer Zunahme von Signalen des Mischlabels A/B.

### Ausführungsbeispiele:

Um die Eigenschaften des erfindungsgemäßen Verfahrens weiterführend zu belegen, wurden zudem die nachfolgend beschriebenen *In-Situ*-Hybridisierungen durchgeführt:

### FISH-Analyse zum Nachweis von multipler numerischer Aberrationen in verschiedenen Zelltypen unter Verwendung der Fünffach-FISH-Sonde "SPEC ERBB2, EGFR. FGFR1, MET & SOX2 FiveCheck™ NG-FISH Probe" der Fa. ZytoVision GmbH

Die Durchführung der FISH erfolgt an 3 bis 5 µm dicken Schnitten von Formalinfixierten Paraffin-eingebetteten (FFPE) Lungen- und Mammakarzinom-Präparaten ohne bzw. mit vorab diagnostizierter ERBB2-Genamplifikation, die auf beschichtete Glasobjektträger aufgezogen und über Nacht bei 58°C gebacken werden.

Zur Entfernung des Paraffins werden die Präparate zunächst für 10 Minuten bei 70 °C auf einer Heizplatte erwärmt und anschließend zweimal für jeweils 10 Minuten bei Raumtemperatur (RT) in 100 % Xylol inkubiert. Danach werden die Präparate mittels einer absteigenden Ethanolreihe (jeweils 5 Minuten bei RT in 96 %, 96 %, 90 %, 70 % vergälltem Ethanol) und Inkubation in Reinstwasser (zweimal jeweils zwei Minuten bei RT) rehydriert. Zur Permeabilisierung der Zellen schließt sich eine Hitzevorbehandlung für 15 Minuten bei 98 °C in Heat Pretratment Solution Citric (ZytoVision GmbH) an, gefolgt von zwei weiteren Inkubationsschritten für 2 Minuten in Reinstwasser bei RT. Die proteolytische Vorbehandlung erfolgt durch Auftropfen von Pepsin-Lösung (Pepsin Solution, ZytoVision GmbH) auf die Präparate und anschließende Inkubation in einer feuchten Kammer bei 37 °C für 25 Minuten. Nach anschließender Inkubation für 5 min in Wash Buffer SSC (ZytoVision Gmb) werden die Präparate dehydriert (jeweils eine Minute bei RT in Reinstwasser, 70 %, 90 %, 96 % Ethanol). Nach Lufttrocknung der Präparate werden jeweils 10 µl der FISH-Sonde ZytoLight SPEC ERBB2, EGFR, FGFR1, MET & SOX2 Five-Check™ NG-FISH Probe (ZytoVision GmbH) mittels Pipette direkt auf die Schnitte aufgetragen.

Bei der Sonde handelt es sich um eine Mischung auf Basis von fünf lokusspezifischen Hybridisierungsonden, wobei die Mischung aus grün-markierten Polynukleotiden (Absorption bei 503 nm und Emission bei 528 nm), die gegen die Region 17q11.2-q12 des ERBB2-Gens, die Region 7p12 des EGFR-Gens, die Region 8p11.23-p11.22 des FGFR1-Gens, die Region 7q31 des MET-Gens und die Region 3q26.3-q27 des SOX2-Gens gerichtet sind sowie aus blau-markierten Polynukleotiden (Absorption bei 426 nm und Emission bei 480 nm), die gegen die Region des EGFR-Gens und des SOX2-Gens gerichtet sind, goldgelb-markierten Polynukleotiden (Absorption bei 532 nm und Emission bei 553 nm), die gegen die Region des FGFR1-Gens gerichtet sind, und rot-markierten Polynukleotiden (Absorption bei 580 nm und Emission bei 599 nm), die gegen die Region des MET-Gens und des SOX2-Gens gerichtet sind, besteht. Anschließend werden Deckgläser luftblasenfrei aufgelegt und die Kanten mit Fixogum (Marabu) versiegelt. Nach Denaturierung der Präparate für zehn Minuten bei 75 °C auf einer Heizplatte wird die Hybridisierung in einer vorgewärmten feuchten Kammer bei 37 °C über Nacht (ungefähr 16 Stunden) in einem Wärmeofen durchgeführt.

Nach Hybridisierung wird das Fixogum entfernt und die Präparate für drei Minuten bei 37 °C in Waschpuffer (1x Wash Buffer A, ZytoVision GmbH) in einer Glasküvette inkubiert. Nach Entfernung der Deckgläser erfolgt eine Stringenzwaschung für jeweils zweimal fünf Minuten bei 37 °C in Waschpuffer (1x Wash Buffer A, ZytoVision GmbH). Anschließend werden die Präparate in einer aufsteigenden Ethanolreihe (jeweils eine Minute bei RT in 70 %, 90 %, 96 %) dehydriert und getrocknet, wobei die Präparate vor direktem Licht geschützt werden. Nach Applikation der Gegenfärbung (20 µl DAPI DuraTect Solution (ZytoVision GmbH)) werden Deckgläser luftblasenfrei aufgelegt und die Präparate lichtgeschützt für mindestens 30 Minuten bei RT inkubiert.

Anschließend erfolgt die Auswertung am Fluoreszenzmikroskop (Axio Scope.A1 mit Beleuchtungseinheit HXP 120 V, Carl Zeiss Microscopy GmbH) unter Verwendung geeigneter Filtersätze (Sp. Green HC mFISH Filterset; Sp. Red HC mFISH Filterset; Sp. Aqua HC mFISH Filterset; ZyGold HC mFISH Filterset (alle AHF Analysentechnik AG)).

Dabei zeigen sich in den Präparaten ohne ERBB2-Amplifikation bei Verwendung des grün-Filters in den Zellkernen jeweils zehn grüne Signale. Unter Verwendung des ZyGold-Filters werden pro Zellkern jeweils zwei goldgelbe-Signale gesehen, deren räumliche Lage mit der von zwei grünen Signalen identisch ist. Unter Verwendung des rot-Filters werden pro Zellkern jeweils vier rote-Signale gesehen, deren räumliche Lage mit der von vier grünen Signalen identisch ist. Unter Verwendung des aqua-Filters werden pro Zellkern jeweils vier aqua-Signale gesehen, deren räumliche Lage mit der von vier grünen Signalen identisch ist sowie bei jeweils zwei Signalen auch identisch mit zwei roten Signalen. Das Signalmuster wird wie folgt interpretiert: Zwei grüne Signale ohne räumlich identische Lokalisation von Signalen einer anderen Farbe identifizieren die beiden ERBB2-Genkopien einer diploiden Zelle. Zwei grüne Signale mit räumlich identischer Lokalisation von zwei aqua-Signalen identifizieren die beiden EGFR-Genkopien, zwei grüne Signale mit räumlich identischer Lokalisation von zwei goldgelben-Signalen identifizieren die beiden FGFR1-Genkopien, zwei grüne Signale mit räumlich identischer Lokalisation von zwei roten-Signalen identifizieren die beiden MET-Genkopien und zwei grüne Signale mit räumlich identischer Lokalisation von zwei roten und zwei aqua-Signalen identifizieren die beiden SOX2-Genkopien.

In den Zellkernen der Präparate mit ERBB2-Amplifikation zeigt sich ein zum vorab beschriebenen Beispiel vergleichbares Signalmuster, mit der Ausnahme, dass neben neun grünen Signalen ein grünes Signalcluster bzw. Signalmuster, bestehend aus ungefähr fünfzehn untrennbar eng beieinanderliegenden Signalen, zu beobachten ist. Dieses grüne Signalmuster ko-lokalisiert nicht mit Signalen einer anderen Farbe und identifizierte somit eine ERBB2-Genamplifikation.

### FISH-Analyse zum Nachweis von Translokationen der ALK- bzw. ROS1-Regionen in verschiedenen Zelltypen unter Verwendung der Vierfach-FISH-Sonde "ZytoLight SPEC ALK & ROS1 Break Apart Single-Mix NG-FISH Probe" der Fa. ZytoVision GmbH

Die Durchführung der FISH erfolgt an 3 bis 5 µm dicken Schnitten von Formalinfixierten Paraffin-eingebetteten (FFPE) Zellen der Zelllinien HeLa (ATCC® CCL-2™), HCC78 (zur Verfügung gestellt von Prof. Schildhaus, Göttingen) und H3122 (zur Verfügung gestellt von Prof. Schildhaus, Göttingen), die auf beschichtete Glasobjektträger aufgezogen und über Nacht bei 58 °C gebacken werden.

Zur Entfernung des Paraffins werden die Präparate zunächst für 10 min bei 70 °C auf einer Heizplatte erwärmt und anschließend zweimal für jeweils 10 min bei Raumtemperatur (RT) in 100 % Xylol inkubiert. Danach werden die Präparate mittels einer absteigenden Ethanolreihe (jeweils fünf min bei RT in 96 %, 96 %, 90 %, 70 % vergälltem Ethanol) und Inkubation in Reinstwasser (zweimal jeweils zwei Minuten bei RT) rehydriert. Zur Permeabilisierung der Zellen schließt sich eine Hitzevorbehandlung für 15 Minuten bei 98 °C in Heat Pretratment Solution Citric (ZytoVision GmbH) an, gefolgt von zwei weiteren Inkubationsschritten für zwei Minuten in Reinstwasser bei RT. Die proteolytische Vorbehandlung erfolgt durch Auftropfen einer Pepsin-Lösung (Pepsin-Solution, ZytoVision GmbH) auf die Präparate und anschließender Inkubation in einer feuchten Kammer bei 37 °C für 15 Minuten. Nach anschließender Inkubation für fünf Minuten in Waschpuffer (Wash Buffer SSC, ZytoVision GmbH) werden die Präparate dehydriert (jeweils eine Minute bei RT in Reinstwasser, 70%, 90%, 96% Ethanol). Nach Lufttrocknung der Präparate werden jeweils 10 µl der FISH-Sonde ZytoLight SPEC ALK & ROS1 Break Apart Single-Mix NG-FISH Probe (ZytoVision GmbH) mittels Pipette direkt auf die Schnitte aufgetragen.

Bei der Sonde handelt es sich um eine Mischung auf Basis von vier lokusspezifischen Hybridisierungsonden, wobei die Mischung aus grün-markierten Polynukleotiden (Absorption bei 503 nm und Emission bei 528 nm), die in 2p23 gegen proximal zur ALK-Bruchpunktregion gelegene Sequenzen und in 6q22 gegen proximal zur ROS1-Bruchpunktregion gelegene Sequenzen gerichtet sind, orangemarkierten Polynukleotiden (Absorption bei 547 nm und Emission bei 572 nm), die in 2p23 gegen distal zur ALK-Bruchpunktregion gelegene Sequenzen und in 6q22 gegen distal zur ROS1-Bruchpunktregion gelegene Sequenzen gerichtet sind, sowie blau-markierten Polynukleotiden (Absorption bei 426 nm und Emission bei 480 nm), die in der Region 6q22 gegen proximal zur ROS1-Bruchpunktregion gelegene Sequenzen gerichtet sind, besteht. Anschließend werden Deckgläser luftblasenfrei aufgelegt und die Kanten mit Fixogum (Marabu) versiegelt. Nach Denaturierung der Präparate über eine Dauer von 10 Minuten bei 75 °C auf einer Heizplatte wird die Hybridisierung in einer vorgewärmten feuchten Kammer bei 37 °C über Nacht (ca. 16 Stunden) in einem Wärmeofen durchgeführt.

Nach Hybridisierung wird das Fixogum entfernt und die Präparate werden für drei Minuten bei 37 °C in Waschpuffer (1x Wash Buffer A, ZytoVision GmbH) in einer Glasküvette inkubiert. Nach Entfernung der Deckgläser erfolgt eine zweimalige Stringenzwaschung für jeweils fünf Minuten bei 37 °C in Waschpuffer (1x Wash Buffer A, ZytoVision GmbH). Anschließend werden die Präparate in einer aufsteigenden Ethanolreihe (jeweils eine Minute bei RT in 70 %, 90 %, 96 % Ethanol) dehydriert und luftgetrocknet, wobei die Proben vor direktem Lichteinfall geschützt werden. Nach Applikation der Gegenfärbung (20 µl DAPI DuraTect Solution, ZytoVision GmbH) werden Deckgläser luftblasenfrei aufgelegt und die Präparate lichtgeschützt für mindestens 30 Minuten bei RT inkubiert.

Anschließend erfolgt die Auswertung am Fluoreszenzmikroskop (Axio Scope.A1 mit Beleuchtungseinheit HXP 120 V, Carl Zeiss Microscopy GmbH) unter Verwendung geeigneter Filtersätze (Dualband Green / Orange-Red Filterset, AHF Analysentechnik; Sp. Aqua HC mFISH Filterset, AHF Analysentechnik). Dabei zeigen sich bei Verwendung des orange/grün-Doppelfilters in der Zellkernen der HeLa-Zelllinie in der Mehrheit der analysierten Kerne jeweils sechs orange/grüne Fusionssignale, einzelne grüne und/oder orange Signale werden nicht gesehen. Unter Verwendung des aqua-Filters werden pro Zellkern jeweils drei aqua-Signale gesehen, deren räumliche Lage mit der von dreien der Fusionssignale identisch ist. Das Signalmuster wird, in Übereinstimmung mit der Literatur, als drei Kopien des ALK-Gens und drei Kopien des ROS1-Gens interpretiert. ALK oder ROS1-Translokationen liegen nicht vor.

In den Zellkernen der Zelllinie H3122, für welche in der Literatur eine Translokation des ALK-Gens beschrieben wird, zeigen sich bei Verwendung des orange/grün-Doppelfilters in der Mehrheit der analysierten Kerne jeweils sieben orange/grüne Fusionssignale und ein einzelnes oranges Signal. Unter Verwendung des aqua-Filters werden pro Zellkern jeweils zwei aqua-Signale gesehen, deren räumliche Lage mit der von zweien der Fusionssignale identisch ist. Das Signalmuster wird, in Übereinstimmung mit der Literatur, als sechs Kopien des ALK-Gens, eines davon von einer Translokation betroffen, und zwei Kopien des ROS1-Gens interpretiert.

In den Zellkernen der Zelllinie HCC78, für welche in der Literatur eine Translokation des ROS1-Gens beschrieben wird, zeigen sich bei Verwendung des orange/grün-Doppelfilters in der Mehrheit der analysierten Kerne jeweils vier orange/grüne Fusionssignale, zwei einzelne orange Signale und zwei einzelne, d.h. davon separate, grüne Signale. Unter Verwendung des aqua-Filters werden pro Zellkern jeweils vier aqua-Signale gesehen, deren räumliche Lage mit zweien der Fusionssignale und den beiden separaten grünen Signalen identisch ist. Das Signalmuster wird, in Übereinstimmung mit der Literatur, als vier Kopien des ROS1-Gens, wobei zwei davon von einer Translokation betroffen sind, und 2 Kopien des ALK-Gens interpretiert

### FISH-Analyse zum Nachweis einer Translokation der ROS1-Region in 6q22 unter Verwendung der Vierfach-FISH-Sonde "ZytoLight SPEC ALK & ROS1 Break Apart Single-Mix NG-FISH Probe" der Fa. ZytoVision GmbH

Es wird eine FISH-Analyse zum Nachweis einer Translokation der ROS1-Region in 6q22 unter Verwendung der Vierfach-FISH-Sonde "ZytoLight SPEC ALK & ROS1 Break Apart Single-Mix NG-FISH Probe" der Fa. ZytoVision GmbH durchgeführt. Bei der Sonde handelt es sich um eine Mischung auf Basis von vier lokusspezifischen Hybridisierungsonden, wobei die Mischung aus grün-markierten Polynukleotiden (Absorption bei 503 nm und Emission bei 528 nm), die in 2p23 gegen proximal zur ALK-Bruchpunktregion gelegene Sequenzen und in 6q22 gegen proximal zur ROS1-Bruchpunktregion gelegene Sequenzen gerichtet sind, orangemarkierten Polynukleotiden (Absorption bei 547 nm und Emission bei 572 nm), die in 2p23 gegen distal zur ALK-Bruchpunktregion gelegene Sequenzen und in 6q22 gegen distal zur ROS1-Bruchpunktregion gelegene Sequenzen gerichtet sind, sowie blau-markierten Polynukleotiden (Absorption bei 426 nm und Emission bei 480 nm), die in der Region 6q22 gegen proximal zur ROS1-Bruchpunktregion gelegene Sequenzen gerichtet sind, besteht.

Bei Verwendung geeigneter Filtersätze erscheinen die Hybridisierungssignale für nicht-rearrangierte ROS1 und/oder ALK-Gene als grün-orange Fluoreszenz-Fusionssignale und für ein rearrangiertes ROS1 und/oder ALK-Gene als ein separates grünes und separates oranges Signal. ROS1 spezifische grüne Signale ko-lokalisieren dabei mit blauen Fluoreszenzsignalen, so dass das nicht-rearrangierte ROS1-Gen sich aus orangen und grün/blauen Fluoreszenz-Mischsignalen zusammensetzt. Die Hybridisierungssignale für das nicht-rearrangierte ALK-Gen erscheinen als grün-orange Fluoreszenz-Fusionssignale ohne Mischsignale mit blauen Fluoreszenzsignalen. Der von einer ROS1-Translokation betroffene 6q22-Lokus ist durch ein separates grünes und ein separates oranges Signal gekennzeichnet. Dabei überlappt sich das separate grüne Signal mit einem blauen Signal. Dieses grün/blaue Mischsignal indiziert ROS1, nicht ALK, als das von der Translokation betroffene Gen. Mit geeigneten Filtersets lässt sich das Signalmuster gut sichtbar machen.

### CISH-Analyse zum Nachweis einer Translokation der ALK-Region in 2p23 unter Verwendung der Vierfach-CISH-Sonde "ZytoDot SPEC ALK & ROS1 Break Apart Single-MIX NG-FISH Probe" der Fa. ZytoVision GmbH

Weiterhin wurde eine CISH-Analyse zum Nachweis einer Translokation der ALK-Region in 2p23 unter Verwendung der Vierfach-CISH-Sonde "ZytoDot SPEC ALK & ROS1 Break Apart Single-MIX NG-FISH Probe" der Fa. ZytoVision GmbH durchgeführt. Bei der Sonde handelt es sich um eine Mischung auf Basis von vier lokusspezifischen Hybridisierungsonden, wobei die Mischung aus Digoxigeninmarkierten Polynukleotiden, die in 2p23 gegen proximal zur ALK-Bruchpunktregion gelegene Sequenzen und in 6q22 gegen proximal zur ROS1-Bruchpunktregion gelegene Sequenzen gerichtet sind, DNP-markierten Polynukleotiden, die in 2p23 gegen distal zur ALK-Bruchpunktregion gelegene Sequenzen und in 6q22 gegen distal zur ROS1-Bruchpunktregion gelegene Sequenzen gerichtet sind, sowie Biotin-markierten Polynukleotiden, die in der Region 6q22 gegen distal zur ROS1-Bruchpunktregion gelegene Sequenzen gerichtet sind, besteht. Der Nachweis der Markierungen erfolgt über primäre (nicht markierte) Antikörper (Anti-DIG/Anti-DNP/Anti-BIO), die von sekundären polymerisierten Enzym-konjugierten Antikörpern (HRP-Polymer/AP-Polymer/beta-GAL) detektiert werden, sowie die enzymatische Umsetzung der Substrate (AP-RED/HRP-GREEN/beta-GAL-BLUE), die zur Entstehung starker, permanenter, roter, grüner und blauer Signale führt, die lichtmikroskopisch z.B. mit einer 40x-Trockenlinse dargestellt werden können.

Diploide bzw. disome Zellkerne ohne Rearrangierungen bzw. Translokationen der ALK bzw. ROS1 Gene zeigen zwei Signale, jeweils bestehend aus einem roten und einem grünen Signal, welche untrennbar dicht beieinander liegen bzw. sich teils überlappen oder vermischen und spezifisch für die beiden Kopien des ALK-Gens sind. Darüber hinaus zeigen sich zwei 2 Signale, welche jeweils aus einem roten, einem grünen und einem blauen Signal bestehen und untrennbar dicht beieinander liegen bzw. sich teils überlappen bzw vermischen und welche spezifisch für die beiden Kopien des ROS1-Gens sind.

Diploide bzw. disome Zellkerne mit Rearrangierungen bzw. Translokationen eines ALK Gens, nicht jedoch der ROS1 Allele, zeigen ein rot-grünes Signal, welches spezifisch für das nicht-rearrangierte ALK-Allel ist. Darüber hinaus zeigen sie ein einzelnes grünes Signal und ein einzelnes, davon separates, rotes Signal, welches spezifisch für ein rearrangiertes ALK-Allel ist. Darüber hinaus zeigen sich zwei rot-grün-blaue Signale, welche spezifisch für die beiden Kopien des ROS1-Gens sind.

Diploide bzw. disome Zellkerne mit Rearrangierungen bzw. Translokationen eines ROS1 Gens, nicht jedoch der ALK Allele, zeigen ein rot-grün-blaues Signal, welches spezifisch für das nicht-rearrangierte ROS1-Allel ist. Darüber hinaus zeigen sie ein einzelnes grünes Signal und ein einzelnes, davon separates, rot-blaues Signal, spezifisch für ein rearrangiertes ROS1-Allel sowie zwei rot-grüne Signale, weiche spezifisch für die beiden Kopien des ALK-Gens sind.

### FISH-Analyse zum Nachweis der Amplifikation der ERBB2-Region unter Verwendung der Fünffach-FISH-Sonde "ZytoLight SPEC ERBB2, EGFR, FGFR1, MET & SOX2 FiveCheck™ NG-FISH Probe" der Fa. ZytoVision

Schließlich wurde eine FISH-Analyse zum Nachweis der Amplifikation der ERBB2-Region unter Verwendung der Fünffach-FISH-Sonde "ZytoLight SPEC ERBB2, EGFR, FGFR1, MET & SOX2 FiveCheck™ NG-FISH Probe" der Fa. ZytoVision durchgeführt. Bei der Sonde handelt es sich um eine Mischung auf Basis von fünf lokusspezifischen Hybridisierungsonden, wobei die Mischung aus grün-markierten Polynukleotiden (Absorption bei 503 nm und Emission bei 528 nm), die gegen die Region 17q11.2-q12 des ERBB2-Gens, die Region 7p12 des EGFR-Gens, die Region 8p11.23-p11.22 des FGFR1-Gens, die Region 7q31 des MET-Gens und die Region 3q26.3-q27 des SOX2-Gens gerichtet sind sowie aus blau-markierten Polynukleotiden (Absorption bei 426 nm und Emission bei 480 nm), die gegen die Region des EGFR-Gens und des SOX2-Gens, goldgelb-markierten Polynukleotiden (Absorption bei 532 nm und Emission bei 553 nm), die gegen die Region des FGFR1-Gens und rot-markierten Polynukleotiden (Absorption bei 580 nm und Emission bei 599 nm), die gegen die Region des MET-Gens und des SOX2-Gens gerichtet sind, besteht.

Bei Verwendung geeigneter Single-Bandpass-Filtersets zeigen sich neun einzelne grüne Signale und ein grünes Signalcluster, welches die Fläche mehrerer einzelner grüner Signale einnimmt, vier blaue Signale, zwei goldgelbe Signale und vier rote Signale.

Durch Überlagerung der Bilder zeigt sich, dass ein einzelnes grünes Signal sowie das grüne Signalcluster nicht mit andersfarbigen Signalen ko-lokalisieren. Bei dem einzelnen grünen Signal handelt es sich um ein nicht-amplifiziertes ERBB2-Gen, das grüne-Signalcluster identifiziert eine ERBB2-Gen-Amplifikation. Ko-lokalisierende grün/blaue Mischsignale identifizieren zwei Kopien des EGFR-Gens, ko-lokalisierende grün/goldgelbe Mischsignale identifizieren zwei Kopien des FGFR1-Gens, ko-lokalisierende grün/rote Mischsignale identifizieren zwei Kopien des MET-Gens und ko-lokalisierende grün/blau/rote Mischsignale identifizieren zwei Kopien des SOX2-Gens.

## Patentansprüche

1. Verfahren zum Nachweis von mindestens zwei voneinander verschiedenen Chromosomenaberrationen mittels *In-Situ*-Hybridisierung durch Detektion von Chromosomen- und/oder DNA-Bereichen in einer biologischen Probe,
**dadurch gekennzeichnet,**
**dass** die *In-Situ*-Hybridisierung als Interphase-*In*-*Situ*-Hybridisierung durchgeführt wird,
**dass** die *In-Situ*-Hybridisierung mit mindestens drei voneinander verschiedenen, mit jeweils einem ersten Detektionslabel markierten, lokusspezifischen Hybridisierungssonden durchgeführt wird, wobei insbesondere zur Erzeugung mindestens eines Mischsignals mindestens eine der lokusspezifischen Hybridisierungssonden mit mindestens einem weiteren, von dem ersten Detektionslabel verschiedenen Detektionslabel, bezogen auf die jeweilige lokusspezifische Hybridisierungssonde, markiert ist, so dass ein Signalmuster erzeugt wird, und
**dass** vorhandene Chromosomenaberrationen anhand des Signalmusters identifiziert und/oder einem Chromosomen- und/oder DNA-Bereich zugeordnet werden,
wobei die Hybridisierungssonden mit den Detektionslabeln direkt markiert sind.

2. Verfahren nach Anspruch 1, wobei die Chromosomenaberrationen voneinander unabhängige Chromosomenaberrationen sind und/oder wobei die Chromosomenaberrationen nicht reziprok sind.

3. Verfahren nach Anspruch 1 oder 2, wobei die Markierung weiterer lokusspezifischer Hybridisierungssonden mit dem mindestens einen weiteren Detektionslabel erfolgt, wobei die mit weiteren Detektionslabeln markierten lokusspezifischen Hybridisierungssonden jeweils voneinander verschiedene Mischsignale in dem Signalmuster erzeugen, und/oder wobei die Markierung weiterer lokusspezifischer Hybridisierungssonden mit dem mindestens einen weiteren Detektionslabel erfolgt, wobei durch jede, mit mindestens einem weiteren Detektionslabel markierte lokusspezifische Hybridisierungssonde in dem Signalmuster ein für einen Chromosomen- und/oder DNA-Bereich spezifisches Mischsignal erzeugt wird; und
wobei mindestens zwei weitere lokusspezifische Hybridisierungssonden mit mindestens einem weiteren, von dem ersten verschiedenen Detektionslabel markiert sind und/oder wobei durch mindestens zwei weitere lokusspezifische Hybridisierungssonden in dem Signalmuster jeweils für einen Chromosomen- und/oder DNA-Bereich spezifische Mischsignale erzeugt werden.

4. Verfahren nach einem der vorangehenden Ansprüche, wobei jeweils zwei lokusspezifische Hybridisierungssonden einen Chromosomenabschnitt flankieren, wobei die jeweils einen Chromosomenabschnitt flankierenden lokusspezifischen Hybridisierungssonden mit voneinander verschiedenen Detektionslabeln markiert sind, so dass durch die jeweils einen Chromosomenabschnitt flankierenden lokusspezifischen Hybridisierungssonden in dem Signalmuster ein Fusionssignal erzeugt wird.

5. Verfahren nach Anspruch 4, wobei mindestens sechs verschiedene lokusspezifische Hybridisierungssonden eingesetzt werden, wobei jeweils zwei lokusspezifische Hybridisierungssonden jeweils einen Chromosomenabschnitt flankieren und
wobei die Markierung weiterer lokusspezifischer Hybridisierungssonden mit mindestens einem weiteren, von dem ersten verschiedenen Detektionslabel derart erfolgt, dass in dem Signalmuster anhand von Fusions- und Mischsignalen jeder flankierte Chromosomenabschnitt und/oder jeder zu detektierende Chromosomen- und/oder DNA-Bereich identifiziert und/oder zugeordnet werden kann.

6. Verfahren nach Anspruch 4 oder 5, wobei
(a) eine mit einem Detektionslabel A markierte erste lokusspezifische Hybridisierungssonde und eine mit einem Detektionslabel B markierte zweite lokusspezifische Hybridisierungssonde einen Chromosomenabschnitt flankieren und in dem mittels In-Situ-Hybridisierung erzeugten Signalmuster ein Fusionssignal A-B erzeugen,
(b) 2 bis 12 weitere lokusspezifische Hybridisierungssonden bis zu sechs weitere Chromosomenabschnitte flankieren, wobei ebenfalls jeweils eine der beiden einen Chromosomenabschnitt flankierenden lokusspezifischen Hybridisierungssonden mit einem Detektionslabel A markiert ist und jeweils eine der beiden einen Chromosomenabschnitt flankierenden lokusspezifischen Hybridisierungssonden mit einem Detektionslabel B markiert ist, so dass die jeweils einen Chromosomenabschnitt flankierenden lokusspezifischen Hybridisierungssonden in dem mittels *In-Situ-*Hybridisierung erzeugten Signalmuster ein Fusionssignal A-B erzeugen und
(c) mindestens eine der lokusspezifischen Hybridisierungssonden mit mindestens einem weiteren Detektionslabel X markiert ist, so dass die mit mindestens einem weiteren Detektionslabel markierten lokusspezifischen Hybridisierungssonden in dem mittels *In-Situ*-Hybridisierung erzeugten Signalmuster Fusions- und Mischsignale A-B/X erzeugen,
wobei sich in dem mittels *In-Situ*-Hybridisierung erzeugten Signalmuster die Fusions- und Mischsignale A-B/X bei Chromosomenaberrationen zu Mischsignalen A/X und/oder B/X verändern und/oder
wobei sich in dem mittels *In-Situ*-Hybridisierung erzeugten Signalmuster die Fusionssignale A-B bei Chromosomenaberrationen zu Einzelsignalen A und/oder B verändern,
so dass Chromosomenaberrationen anhand des mittels der *In-Situ*-Hybridisierung erzeugten Signalmusters einem Chromosomen- und/oder DNA-Bereich und/oder einem durch zwei lokusspezifische Hybridisierungssonden flankierten Chromosomenabschnitt zugeordnet werden;
wobei in einem ersten Schritt die durch die ersten Detektionslabel erzeugten Fusionssignale detektiert und/oder analysiert werden und in einem nachfolgenden Schritt bei Auftreten von Einzelsignalen eine Detektion und/oder Analyse der Mischsignale und deren Zuordnung zu den detektierten Chromosomen- und/oder DNA-Bereichen erfolgt und/oder wobei die Detektion des Signalmusters mittels computergestützter Analyse erfolgt.

7. Verfahren nach einem der vorangehenden Ansprüche, wobei die lokusspezifischen Hybridisierungssonden jeweils durch eine Vielzahl von Nukleinsäurefragmenten ("Sondenfragmenten") gebildet werden, welche jeweils den zu detektierenden Chromosomen- und/oder DNA-Bereich abdecken

8. Verfahren nach einem der vorangehenden Ansprüche,
(i) wobei einzelne Nukleinsäurefragmente einer hybridisierungsspezifischen Sonde mit nur einem Detektionslabel markiert sind, und
(ii) wobei einzelne Nukleinsäurefragmente einer hybridisierungsspezifischen Sonde mit mehreren, voneinander verschiedenen Detektionslabeln markiert sind,
wobei auch Kombinationen der Möglichkeiten (i) und (ii) zu Mischsignalen führen.

9. Verfahren nach einem der vorangehenden Ansprüche, wobei die *In-Situ-*Hybridisierung unter direkter Markierung der Hybridisierungssonden erfolgt und wobei die *In-Situ*-Hybridisierung unter Markierung der Hybridisierungssonden mit Fluoreszenzfarbstoffen erfolgt.

10. Zusammensetzung zum Nachweis von mindestens zwei voneinander verschiedenen Chromosomenaberrationen mittels *In-Situ*-Hybridisierung, wobei die Zusammensetzung mindestens drei mit jeweils einem ersten Detektionslabel markierte, lokusspezifische Hybridisierungssonden umfasst, und wobei mindestens eine der lokusspezifischen Hybridisierungssonden mit mindestens einem weiteren, von dem ersten verschiedenen Detektionslabel, bezogen auf die jeweilige lokusspezifische Hybridisierungssonde, markiert ist,
wobei die Hybridisierungssonden mit den Detektionslabeln direkt markiert sind.

11. Zusammensetzung zur Verwendung bei der Diagnose und/oder Prognose von im Zusammenhang mit Chromosomenaberrationen stehenden Erkrankungen, wobei die Erkrankungen ausgewählt sind aus der Gruppe bestehend aus Malignomen, vorzugsweise Karzinomen, Sarkomen und/oder Leukämien,
wobei die Zusammensetzung mindestens drei voneinander verschiedene, mit jeweils einem ersten Detektionslabel markierte, lokusspezifische Hybridisierungssonden umfasst, und wobei mindestens eine der lokusspezifischen Hybridisierungssonden mit mindestens einem weiteren, von dem ersten verschiedenen Detektionslabel, bezogen auf die jeweilige lokusspezifische Hybridisierungssonde, markiert ist,
wobei die Hybridisierungssonden mit den Detektionslabeln direkt markiert sind.

12. Verwendung einer Zusammensetzung nach Anspruch 10 zum Nachweis von Chromosomenaberrationen mittels *In-Situ*-Hybridisierung, insbesondere mittels des Verfahrens nach einem der Ansprüche 1 bis 9.

13. Verwendung von mindestens drei voneinander verschiedenen, mit jeweils einem ersten Detektionslabel markierten, lokusspezifischen Hybridisierungssonden, wobei mindestens eine der lokusspezifischen Hybridisierungssonden mit mindestens einem weiteren, von dem ersten verschiedenen Detektionslabel, bezogen auf die jeweilige lokusspezifische Hybridisierungssonde, markiert ist, vorzugsweise im Rahmen eines Verfahrens nach einem der Ansprüche 1 bis 9, bei der Diagnose und/oder Prognose von im Zusammenhang mit Chromosomenaberrationen stehenden Erkrankungen, insbesondere Malignomen, vorzugsweise Karzinomen, Sarkomen und/oder Leukämien, besonders bevorzugt Lungentumoren, Lymphomen, Leukämien, Sarkomen, Mammakarzinomen und/oder Darmkrebs.

14. Kit zum Nachweis von mindestens zwei voneinander verschiedenen Chromosomenaberrationen mittels *In-Situ*-Hybridisierung, umfassend mindestens drei voneinander verschiedene, mit jeweils einem ersten Detektionslabel markierte, lokusspezifische Hybridisierungssonden, wobei mindestens eine der lokusspezifischen Hybridisierungssonden mit mindestens einem weiteren, von dem ersten verschiedenen Detektionslabel, bezogen auf die jeweilige Hybridisierungssonde, markiert ist,
wobei die Hybridisierungssonden mit den Detektionslabeln direkt markiert sind,

15. Kit nach Anspruch 14, wobei die mindestens drei voneinander verschiedenen lokusspezifischen Hybridisierungssonden in einer gemeinsamen Zusammensetzung vorliegen.

## Claims

1. A method for detection of at least two chromosome abnormalities that are different from one another, by means of in situ hybridization, by means of detection of chromosome regions and/or DNA regions in a biological sample,
**characterized in that**
the in situ hybridization is carried out as interphase/in situ hybridization,
that the in situ hybridization is carried out with at least three locus-specific hybridization probes that are different from one another and marked with a first detection label, in each instance, wherein particularly for generation of at least one mixed signal, at least one of the locus-specific hybridization probes is marked with at least one further detection label, different from the first detection label, with reference to the respective locus-specific hybridization probe, so that a signal pattern is generated, and
that existing chromosome abnormalities are identified using the signal pattern and/or assigned to a chromosome region and/or DNA region,
wherein the hybridization probes are labeled directly with the detection labels.

2. The method according to claim 1, wherein the chromosome abnormalities are chromosome abnormalities that are independent of one another and/or wherein the chromosome abnormalities are not reciprocal.

3. The method according to claim 1 or 2, wherein marking of further locus-specific hybridization probes takes place with the at least one further detection label, wherein the locus-specific hybridization probes that are marked with further detection labels generate mixed signals that are different from one another, in each instance, in the signal pattern, and/or wherein marking of further locus-specific hybridization probes with the at least one further detection label takes place, wherein a mixed signal specific for a chromosome region and/or DNA region is generated by every locus-specific hybridization probe in the signal pattern that is marked with at least one further detection label; and
wherein at least two further locus-specific hybridization probes are marked with at least one further detection label that is different from the first, and/or wherein mixed signals specific for a chromosome region and/or DNA region are generated by means of at least two further locus-specific hybridization probes in the signal pattern.

4. The method according to one of the preceding claims, wherein two locus-specific hybridization probes, in each instance, flank a chromosome segment wherein the locus-specific hybridization probes that flank a chromosome segment are marked with detection labels that are different from one another, so that a fusion signal is generated in the signal pattern by means of the locus-specific hybridization probes that flank a chromosome segment, in each instance.

5. The method according to claim 4, wherein at least six different locus-specific hybridization probes are used, wherein two locus-specific hybridization probes, in each instance, flank a chromosome segment in each instance and
wherein marking of further locus-specific hybridization probes with at least one further detection label that is different from the first takes place in such a manner that in the signal pattern, each flanked chromosome segment and/or each chromosome region and/or DNA region to be detected can be identified and/or assigned, using fusion signals and mixed signals.

6. The method according to claim 4 or 5, wherein
(a) a first locus-specific hybridization probe marked with a detection label A and a second locus-specific hybridization probe marked with a detection label B flank a chromosome segment and generate a fusion signal A-B in the signal pattern generated by means of in situ hybridization,
(b) 2 to 12 further locus-specific hybridization probes flank up to six further chromosome segments wherein also, in each instance, one of the two locus-specific hybridization probes that flank a chromosome segment is marked with a detection label A and, in each instance, one of the two locus-specific hybridization probes that flank a chromosome segment is marked with a detection label B, so that the locus-specific hybridization probes that flank a chromosome segment in each instance, generate a fusion signal A-B in the signal pattern generated by means of in situ hybridization, and
(c) at least one of the locus-specific hybridization probes is marked with at least one further detection label X, so that the locus-specific hybridization probes marked with at least one further detection label generate fusion signals and mixed signals A-B/X in the signal pattern generated by means of in situ hybridization,
wherein the fusion signals and mixed signals A-B/X at chromosome abnormalities change to mixed signals A/X and/or B/X in the signal pattern generated by means of in situ hybridization, and/or
wherein the fusion signals A-B at chromosome abnormalities change to single signals A and/or B in the signal pattern generated by means of in situ hybridization,
so that chromosome abnormalities are assigned to a chromosome region and/or DNA region and/or to a chromosome segment flanked by two locus-specific hybridization probes, using the signal pattern generated by means of the in situ hybridization;
wherein in a first step, the fusion signals generated by the first detection labels are detected and/or analyzed, and, in a subsequent step, if single signals occur, detection and/or analysis of the mixed signals and their assignment to the detected chromosome regions and/or DNA regions takes place, and/or wherein detection of the signal pattern takes place by means of computer-assisted analysis.

7. The method according to one of the preceding claims, wherein the locus-specific hybridization probes are formed, in each instance, by a plurality of nucleic acid fragments ("probe fragments"), which each cover the chromosome region and/or DNA region to be detected.

8. The method according to one of the preceding claims,
(i) wherein individual nucleic acid fragments of a hybridization-specific probe are marked with only one detection label and
(ii) wherein individual nucleic acid fragments of a hybridization-specific probe are marked with multiple detection labels that are different from one another,
wherein also combinations of the possibilities (i) and (ii) lead to mixed signals.

9. The method according to one of the preceding claims, wherein the in situ hybridization takes place with direct marking of the hybridization probes and wherein the in situ hybridization takes place with marking of the hybridization probes with fluorescence dyes.

10. A composition for detection of at least two chromosome abnormalities that are different from one another by means of in situ hybridization wherein the composition comprises at least three locus-specific hybridization probes that are different from one another and marked with a first detection label, in each instance, and wherein at least one of the locus-specific hybridization probes is marked with a further detection label that is different from the first, with reference to the respective locus-specific hybridization probe,
wherein the hybridization probes are labeled directly with the detection labels.

11. A composition for use in diagnosis and/or prognosis of illnesses that are connected with chromosome abnormalities wherein the illnesses are selected from the group consisting of malignancies, preferably carcinomas, sarcomas and/or leukemias,
wherein the composition comprises at least three locus-specific hybridization probes that are different from one another and marked with a first detection label, in each instance, and wherein at least one of the locus-specific hybridization probes is marked with at least one further detection label, different from the first, with reference to the respective locus-specific hybridization probe,
wherein the hybridization probes are labeled directly with the detection labels.

12. Use of a composition according to claim 10, for detection of chromosome abnormalities by means of in situ hybridization particularly by means of the method according to one of claims 1 to 9.

13. Use of at least three locus-specific hybridization probes that are different from one another, each marked with a first detection label, wherein at least one of the locus-specific hybridization probes is marked with at least one further detection label, different from the first, with reference to the respective locus-specific hybridization probe, preferably within the scope of a method according to one of claims 1 to 9, for diagnosis and/or prognosis of illnesses connected with chromosome abnormalities, particularly malignancies, preferably carcinomas, sarcomas and/or leukemias, particularly preferentially lung tumors, lymphomas, leukemias, sarcomas, mamma carcinomas and/or colon cancer.

14. A kit for detection of at least two chromosome abnormalities that are different from one another by means of in situ hybridization comprising at least three locus-specific hybridization probes that are different from one another, each marked with a first detection label, wherein at least one of the locus-specific hybridization probes is marked with at least one further detection label that is different from the first, with reference to the respective hybridization probe,
wherein the hybridization probes are labeled directly with the detection labels.

15. Kit according to claim 14, wherein the at least three locus-specific hybridization probes that are different from one another are present in a common composition.

## Revendications

1. Procédé de détection d'au moins deux aberrations chromosomiques différentes l'une de l'autre au moyen d'une hybridation *in situ* par détection de régions chromosomiques et/ou de régions d'ADN dans un échantillon biologique,
**caractérisé en ce que**
l'hybridation *in situ* est réalisée comme hybridation *in situ* en interphase,
l'hybridation *in situ* est réalisée avec au moins trois sondes d'hybridation spécifiques du locus différentes l'une de l'autre et respectivement marquées avec un premier marqueur de détection, l'une au moins des sondes d'hybridation spécifiques du locus étant, notamment pour la production d'au moins un signal mixte, marquée avec au moins un marqueur de détection supplémentaire différent du premier marqueur de détection de la sonde d'hybridation spécifique du locus en question, de sorte à produire un signal de référence, et **en ce que**
des aberrations chromosomiques peuvent, au moyen du signal de référence, être identifiées et/ou attribuées à une région chromosomique et/ou à une région d'ADN, les sondes d'hybridation étant directement marquées avec les marqueurs de détection.

2. Procédé selon la revendication 1, les aberrations chromosomiques étant des aberrations indépendantes les unes des autres et/ou les aberrations chromosomiques étant non réciproques.

3. Procédé selon la revendication 1 ou 2, le marquage de sondes d'hybridation spécifiques du locus supplémentaires étant effectué avec le (les) marqueur(s) de détection supplémentaire(s), les sondes d'hybridation spécifiques du locus marquées avec des marqueurs de détection supplémentaires produisant dans le signal de référence des signaux mixtes respectivement différents les uns des autres, et/ou le marquage de sondes d'hybridation spécifiques du locus supplémentaires étant effectué avec le (les) marqueur(s) de détection supplémentaire(s), un signal mixte spécifique d'une région chromosomique et/ou d'une région d'ADN étant produit dans le signal de référence par chaque sonde d'hybridation spécifique du locus marquée avec au moins un marqueur de détection supplémentaire, et
deux autres sondes d'hybridation spécifiques du locus au moins étant marquées avec au moins un marqueur de détection différent du premier et/ou des signaux mixtes respectivement spécifiques d'une région chromosomique et/ou d'une région d'ADN étant produit dans le signal de référence par au moins deux autres sondes d'hybridation spécifiques du locus.

4. Procédé selon l'une des revendications précédentes, deux sondes d'hybridation spécifiques du locus encadrant respectivement un segment de chromosome et ces sondes étant marquées avec des marqueurs de détection différents les uns des autres de sorte que les sondes d'hybridation spécifiques du locus qui encadrent respectivement un segment de chromosome produisent un signal de fusion dans le signal de référence.

5. Procédé selon la revendication 4 mettant en oeuvre au moins six sondes d'hybridation spécifiques du locus différentes qui encadrent respectivement deux par deux un segment de chromosome, et
le marquage d'autres sondes d'hybridation spécifiques du locus avec au moins un marqueur de détection différent du premier étant effectué de façon telle que chaque segment de chromosome encadré et/ou chaque région chromosomique et/ou région d'ADN à détecter peut, dans le signal de référence, être identifié et/ou attribué à l'aide de signaux de fusion et de signaux mixtes.

6. Procédé selon la revendication 4 ou 5,
(a) une première sonde d'hybridation spécifique du locus marquée avec un marqueur de détection A et une seconde sonde d'hybridation spécifique du locus marquée avec un marqueur de détection B encadrent un segment de chromosome et produisent un signal de fusion A-B dans le signal de référence produit par hybridation *in situ,*
(b) 2 à 12 autres sondes d'hybridation spécifiques du locus encadrent jusqu'à six autres segments de chromosome, l'une des deux sondes d'hybridation spécifiques du locus encadrant respectivement un segment de chromosome étant également marquée avec un marqueur de détection A et l'autre des deux sondes d'hybridation spécifiques du locus encadrant le segment de chromosome étant marquée avec un marqueur de détection B, de sorte que les sondes d'hybridation spécifiques du locus qui encadrent respectivement un segment de chromosome produisent un signal de fusion A-B dans le signal de référence produit par hybridation *in situ,* et
(c) l'une au moins des sondes d'hybridation spécifiques du locus est marquée avec au moins un autre marqueur de détection X, de sorte à ce qu'elle produise des signaux de fusion et des signaux mixtes A-B/X dans le signal de référence produit par hybridation *in situ,*
les signaux de fusion et signaux mixtes A-B/X produits dans le signal de référence produit par hybridation *in situ* étant, en cas d'aberrations chromosomiques, modifiés en signaux mixtes A/X et/ou B/X, et/ou
les signaux de fusion A-B produits dans le signal de référence produit par hybridation *in situ* étant, en cas d'aberrations chromosomiques, modifiés en signaux individuels A et/ou B,
de sorte que les aberrations chromosomiques soient, au moyen du signal de référence produit par hybridation *in situ,* attribuées à une région chromosomique et/ou à une région d'ADN et/ou à un segment de chromosome encadré par deux sonde d'hybridation spécifique du locus;
les signaux de fusion produits par les premiers marqueurs de détection étant détectés et/ou analysés lors d'une première étape, une étape suivante consistant alors, en cas d'apparition de signaux individuels, à détecter et/ou analyser les signaux mixtes et à les attribuer aux régions chromosomiques et/ou d'ADN détectées, et/ou la détection du signal de référence étant effectuée par analyse assistée par ordinateur.

7. Procédé selon l'une des revendications précédentes, les sondes d'hybridation spécifiques du locus étant formées par une pluralité de fragments d'acide nucléique (« fragments de sonde ») qui couvrent respectivement la région chromosomique et/ou d'ADN à détecter.

8. Procédé selon l'une des revendications précédentes,
(i) certains fragments d'acide nucléique d'une sonde d'hybridation spécifique n'étant marqués qu'avec un seul marqueur de détection et
(ii) certains fragments d'acide nucléique d'une sonde d'hybridation spécifique étant marqués avec plusieurs marqueurs de détection différents les uns des autres,
des combinaisons des possibilités (i) et (ii) produisant également des signaux mixtes.

9. Procédé selon l'une des revendications précédentes, l'hybridation *in situ* ayant lieu avec un marquage direct des sondes d'hybridation et l'hybridation *in situ* ayant lieu avec un marquage des sondes d'hybridation à l'aide de colorants fluorescents.

10. Composition pour la détection d'au moins deux aberrations chromosomiques différentes l'une de l'autre au moyen d'une hybridation *in situ,* la composition comprenant au moins au moins trois sondes d'hybridation spécifiques du locus respectivement marquées avec un premier marqueur de détection, l'une au moins des sondes d'hybridation spécifiques du locus étant marquée avec au moins un marqueur de détection supplémentaire différent du premier marqueur de détection de la sonde d'hybridation spécifique du locus en question,

11. Composition à utiliser pour le diagnostic et/ou le pronostic de maladies en relation avec des aberrations chromosomiques, les maladies étant choisies dans le groupe formé par les affections malignes, de préférence les carcinomes, les sarcomes et/ou les leucémies,
la composition comprenant au moins au moins trois sondes d'hybridation spécifiques du locus différentes l'une de l'autre et respectivement marquées avec un premier marqueur de détection, l'une au moins des sondes d'hybridation spécifiques du locus étant marquée avec au moins un marqueur de détection supplémentaire différent du premier marqueur de détection de la sonde d'hybridation spécifique du locus en question,
les sondes d'hybridation étant directement marquées avec les marqueurs de détection.

12. Utilisation d'une composition selon la revendication 10 pour la détection d'aberrations chromosomiques au moyen d'une hybridation *in situ,* notamment au moyen du procédé selon l'une des revendications 1 à 9.

13. Utilisation d'au moins trois sondes d'hybridation spécifiques du locus différentes l'une de l'autre et respectivement marquées avec un premier marqueur de détection, l'une au moins des sondes d'hybridation spécifiques du locus étant marquée avec au moins un marqueur de détection supplémentaire différent du premier marqueur de détection de la sonde d'hybridation spécifique du locus en question, de préférence dans le cadre d'un procédé selon l'une des revendications 1 à 9, pour le diagnostic et/ou le pronostic de maladies en relation avec des aberrations chromosomiques, notamment des affections malignes, de préférence les carcinomes, les sarcomes et/ou les leucémies, et de préférence particulière des tumeurs du poumon, des lymphomes, des leucémies, des sarcomes, des cancers du sein et/ou du côlon.

14. Kit de détection d'au moins deux aberrations chromosomiques différentes l'une de l'autre au moyen d'une hybridation *in situ,* comprenant au moins au moins trois sondes d'hybridation spécifiques du locus différentes l'une de l'autre et respectivement marquées avec un premier marqueur de détection, l'une au moins des sondes d'hybridation spécifiques du locus étant marquée avec au moins un marqueur de détection supplémentaire différent du premier marqueur de détection de la sonde d'hybridation spécifique du locus en question,
les sondes d'hybridation étant directement marquées avec les marqueurs de détection.

15. Kit selon la revendication 14, les trois sondes d'hybridation spécifiques du locus différentes l'une de l'autre qui le composent au moins formant une composition commune.
